Europäisches Patentamt

European Patent Office

Office européen des brevets

(19)

(11) Veröffentlichungsnummer: **0 181 578 B1**

(12) **EUROPÄISCHE PATENTSCHRIFT**

(45) Veröffentlichungstag der Patentschrift: **04.09.91**

(21) Anmeldenummer: **85113908.9**

(22) Anmeldetag: **31.10.85**

(51) Int. Cl.5: **C07K 7/10, C12P 21/02, A61K 37/02, //C12N1/20, (C12P21/02,C12R1:45), (C12N1/20,C12R1:45)**

(54) Antibiotisches Polypeptid, Verfahren zu seiner Herstellung und seine Verwendung.

(30) Priorität: **06.11.84 DE 3440423**
**01.07.85 DE 3523478**

(43) Veröffentlichungstag der Anmeldung:
**21.05.86 Patentblatt 86/21**

(45) Bekanntmachung des Hinweises auf die Patenterteilung:
**04.09.91 Patentblatt 91/36**

(84) Benannte Vertragsstaaten:
**AT BE CH DE FR GB IT LI LU NL SE**

(56) Entgegenhaltungen:

**CHEMICAL ABSTRACTS, Band 79, Nr. 21, 26. November 1973, Seite 436, Zusammenfassung Nr. 126777h, Columbus, Ohio, US; E. GROSS et al.: "Subtilin. VI. Structure of subtilin", & HOPPE-SEYLER'S Z. PHYSIOL. CHEM. 1973, 354(7), 810-12**

(73) Patentinhaber: **Dr. Karl Thomae GmbH**
**Postfach 1755**
**W-7950 Biberach (Riss)(DE)**

(72) Erfinder: **Werner, Rolf-Günther, Dr. Dipl.-Mikrob**
**Beim Forhäldele 18**
**W-7950 Biberach 1(DE)**
Erfinder: **Zähner, Hans, Prof. Dr.**
**Im Hopfengarten 13**
**W-7400 Tübingen(DE)**
Erfinder: **Jung, Günther, Prof. Dr.**
**Ob der Grafenhalde 5**
**W-7400 Tübingen(DE)**
Erfinder: **Allgaier, Hermann, Dr. Dipl.-Chem.**
**Köhlesrain 105**
**W-7950 Biberach(DE)**
Erfinder: **Schneider, Ursula**
**Schumannstrasse 3**
**W-7255 Rutesheim(DE)**

THE JOURNAL OF THE AMERICAN CHEMICAL
SOCIETY, Band 93, Nr. 18, 8. September 1971,
Seiten 4634-4635; E. GROSS et al.: "The
structure of nisin"

TETRAHEDRON LETTERS, Band 26, Nr. 5,
1985, Seiten 665-668, Pergamon Press Ltd,
GB; T. WAKAMIYA et al.: "The structure of
ancovenin, a new peptide inhibitor of angiotensin I converting enzyme"

**Beschreibung**

Die Erfindung betrifft ein antibiotisch wirkendes Polypeptid, in der Folge Epidermin genannt, ein Verfahren zu seiner Herstellung, mittels eines gegen diese Substanz resistenten neuen Stammes von Staphylococcus epidermidis, den neuen resistenten Stamm, den Wirkstoff Epidermin enthaltende antibiotische Zubereitungsformen und die Verwendung des Wirkstoffes zur Bekämpfung von infektiösen Erkrankungen.

Durch die EP-A-0 027 710 ist es bekannt, daß ein besonderer Stamm des Staphylococcus epidermidis, nämlich Staphylococcus epidermidis NCIB 11536 (hinterlegt bei der National Collection of Industrial Bacteria, Aberdeen) ein niedermolekulares, antibiotisch wirkendes Polypeptid mit einer breiten Wirkung auf grampositive Keime erzeugt, welches Bakterienzellen lysiert.

Es ist ferner bekannt, daß der Stamm Staphylococcus epidermidis MF 205 (S.M. Taylor et al., Int. J.Mass Spectrom. and Ion Physics 48, 161-164, 1983) ebenfalls ein Oligopeptid mit Wirkung gegen grampositive Bakterien produziert. Aus der Veröffentlichung ist es nicht ersichtlich, ob dieser Stamm mit dem oben erwähnten Stamm NCIB 11536 identisch ist.

Es wurde nun gefunden, daß eine resistente Mutante des Staphylococcus epidermidis, die am 26.10.1984 bei der "Deutsche Sammlung von Mikroorganismen" unter der Nummer DSM 3095, hinterlegt wurde und die mit dem obenerwähnten Stamm NCIB 11536 verwandt ist, nach einem modifizierten Herstellungs- und Aufarbeitungsverfahren das ähnliche antibiotisch, vorzugsweise auf grampositive Keime, wirkende Polypeptid Epidermin erzeugt, welches beim Vergleich mit den Produkten aus Staphylococcus epidermidis NCIB 11536 und MF 205 unter anderem erhebliche Unterschiede in der Aminosäurenzusammensetzung aufweist:

Vergleich der Bausteine:

| | Epidermin | Produkte von Staph.epidermidis | |
| --- | --- | --- | --- |
| | | MF 205 | NCIB 11536 |
| Asn | 1 | - | - |
| Asx | - | 2 | 1 |
| Glx | - | 3 | 1 |
| Pro | 1 | 1 | 1 |
| Gly | 2 | 2 | 2 |
| Ala | 2 | 2 | 1 |
| Ile | 2 | 2 | 1 |
| Phe | 2 | 2 | 1 |
| Lys | 2 | 2 | 1 |
| Lan | 2 | - | - |
| ß-Me-Lan | 1 | - | - |
| Dhb | 1 | - | - |
| Tyr | 1 | 1 | |
| S-(2-Aminovinyl)-D-cystein | 1 | - | - |
| X | - | 1-2 | - |

Dhb entspricht der α,ß-Dehydroaminobuttersäure, Asx und Glx bedeuten Asn/Asp bzw. Gln/Glu.

Nach Totalhydrolyse von Epidermin mittels 6N Salzsäure (18 Stunden bei 110°C) lieferte die quantitative Aminosäurenanalyse durch Ionenaustauscherchromatographie (Standardprogramm mit dem Gerät Biotronik LC 6000 E) folgende α-Aminosäuren-Zusammensetzung:

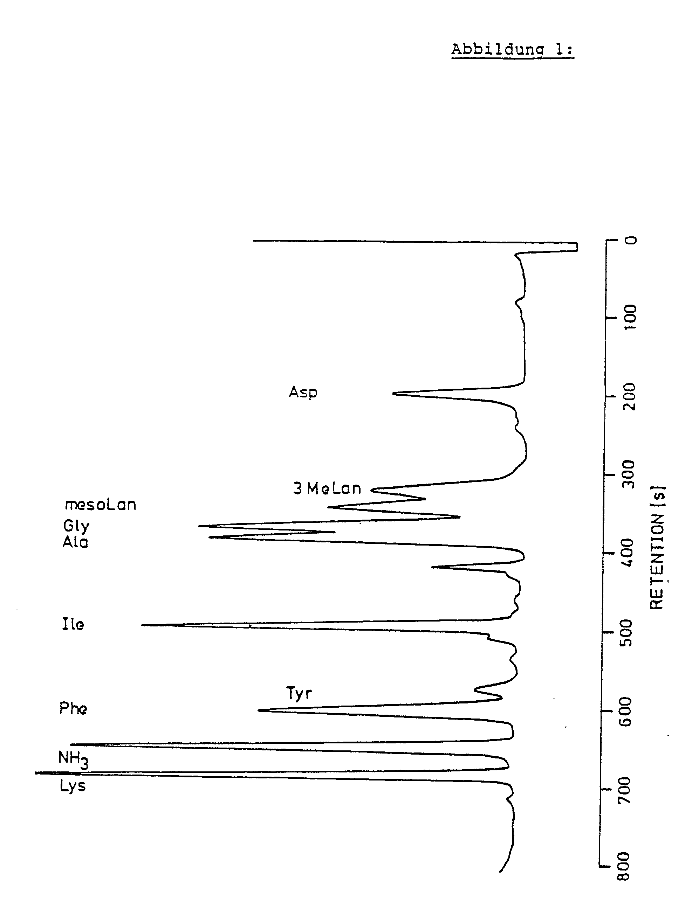

Asp (1,00), Pro (0,95), Gly (2,09), Ala (2,03), Ile (2,03), Tyr (0,30), Phe (2,02) und Lys (2,00) (vgl. Abb. 1). Durch Zugabe von Thioglykolsäure erhöhte sich der Tyrosingehalt des Totalhydrolysats auf den weitgehend stöchiometrischen Wert von 0,93.

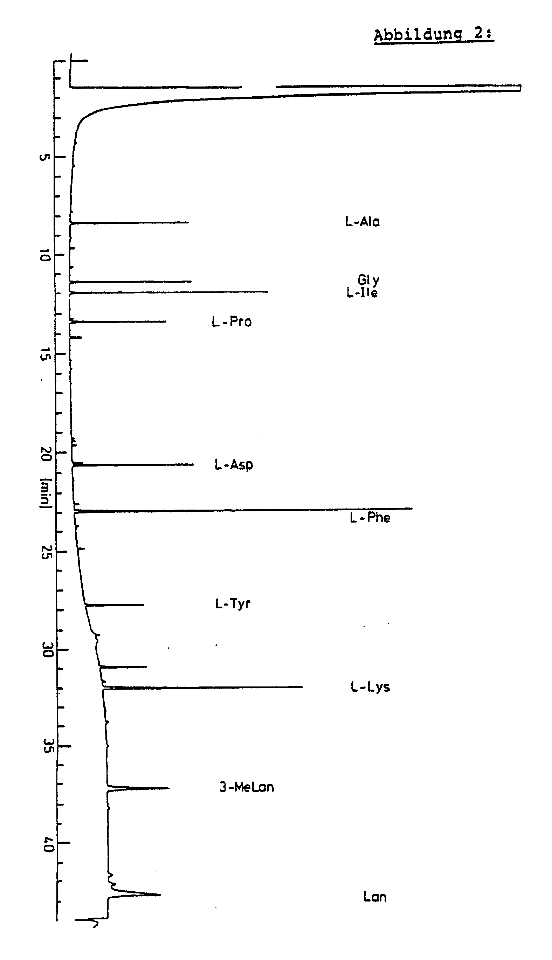

Die Konfiguration der Proteinaminosäuren wurde durch Gaschromatographie der Pentafluoropropionyl-aminosäure-n-propylester aus dem Totalhydrolysat ermittelt. Die Trennung erfolgte an der chiralen stationären Phase L-Valin-tert.-butylamid-polysiloxan (Chirasil-Val). Durch Vergleich mit einer Testmischung von Aminosäuren bekannter Konfiguration konnte den oben angeführten Bausteinen die L-Konfiguration zugeordnet werden (vgl. Abb. 2).

Zu diesen Proteinaminosäuren kommen noch folgende Nichtproteinaminosäuren: Lanthionin (2), β-Methyllanthionin (1) und α,β-Dehydroaminobuttersäure (1); hierbei besitzt Lanthionin die meso- und β-Methyllanthionin die 2S,3S,6R-Konfiguration. Zusätzlich enthält Epidermin den Baustein S-(2-Aminovinyl)-D-cystein.

Die neue Verbindung Epidermin kann durch folgende Angaben charakterisert werden:

1. Natur: farbloses Pulver

2. Löslichkeit: Sehr gut löslich in Mischungen aus Wasser/Eisessig bzw. Methanol/Eisessig, löslich in niederen Alkoholen, unlöslich in Chloroform, Aceton, Diethylether, Petrolether.

3. Farbreaktion auf Kieselgelplatten: Ninhydrin, Chlor/TDM (TDM = 4,4'-Bis-(dimethylamino)-diphenylmethan), Orcin/Schwefelsäure, Anisaldehyd/Schwefelsäure. Zerstörungsfreier Nachweis im UV-Licht bei 254 nm und durch Sprühen mit Wasser.

4. Dünnschichtchromatographie: Es wurden Kieselgelfertigplatten 60 $F_{254}$ (Merck) verwendet.

System A: Chloroform/Methanol/17% Ammoniak (2/2/1),      RF = 0,73

System B: Chloroform/Methanol/17% Ammoniak (70/35/10)      RF = 0,30

System C: n-Butanol/Eisessig/Wasser (4/1/1)      RF = 0,05

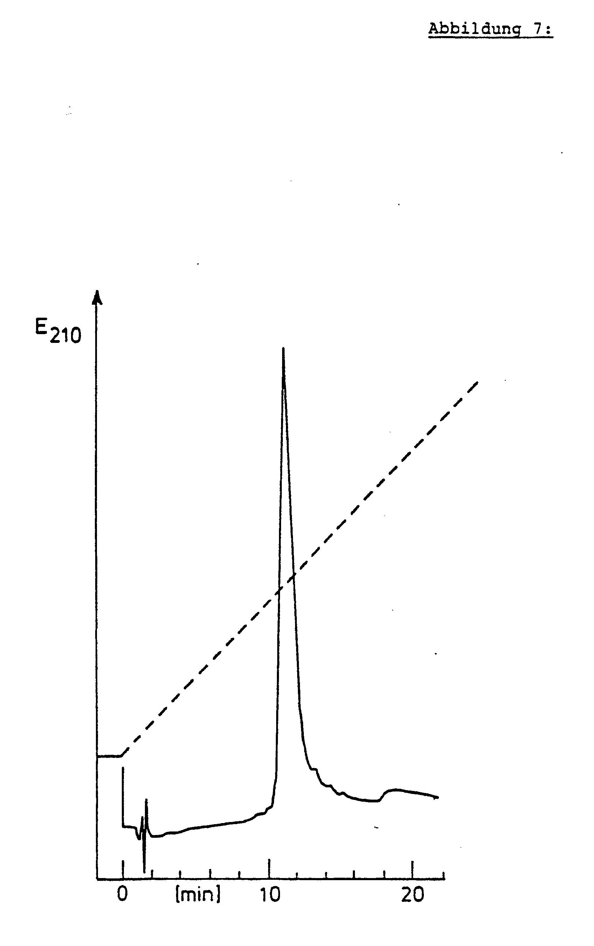

5. HPLC : siehe Abb. 7.

6. Stabilität: Stabil von pH = 2 bis pH = 7, bei höheren pH-Werten tritt starker Aktivitätsabfall ein.

7. Molmasse: im Bereich von 2160. Je nach Isolierungsmethoden können als Anionen, z.B. Chlorid, Acetat oder Phosphat, enthalten sein.

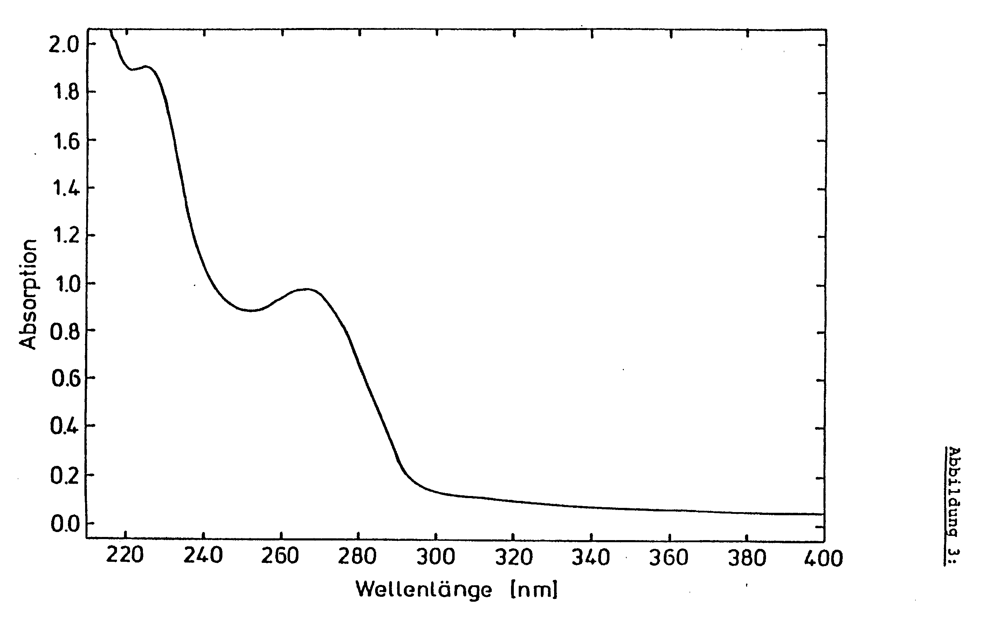

8. Ultraviolettabsorptionsspektrum: In wässriger Lösung langwelliges Maximum bei 267 nm (Abb. 3).

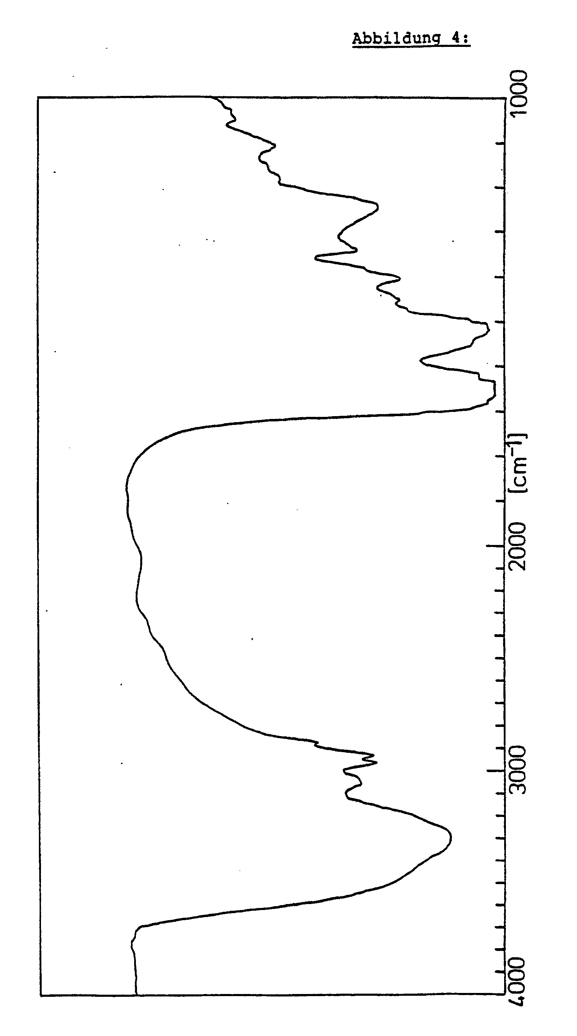

9. Infrarotabsorptionsspektrum: IR-Spektrum einer Probe in einer Kaliumbromidtablette (Abb. 4).

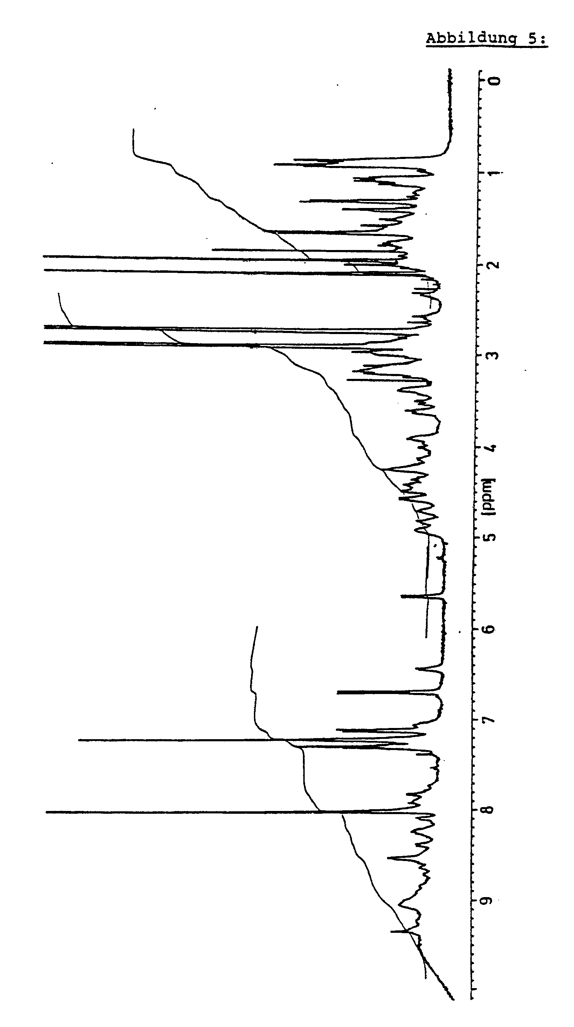

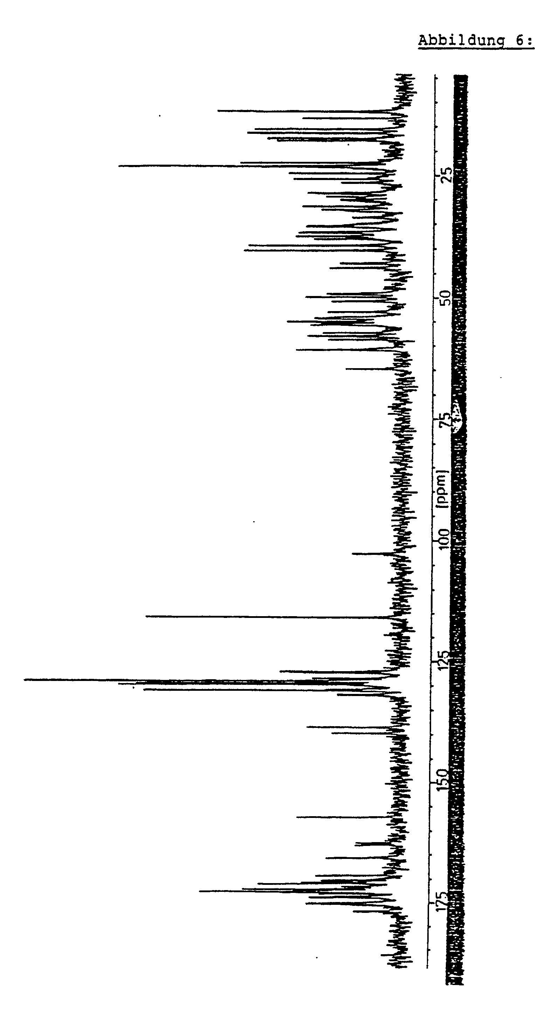

10. Kernmagnetische Resonanzspektren $^1$H-NMR Spektrum: Abb. 5, $^{13}$C-NMR-Spektrum: Abb. 6

Geeignete Fragmente zur Sequenzierung des Epidermins wurden durch tryptische Spaltung erhalten. Die Umsetzung mit Trypsin führte in kurzer Zeit zum Verlust der antibiotischen Aktivität. Makroskopisch beobachtete man bei der tryptischen Spaltung einen gallertartigen weißen Niederschlag, welcher durch Zentrifugation abgetrennt werden konnte. Der Überstand wurde lyophilisiert und an Sephadex G-25 (Dextrangel der Firma Pharmacia) in 1%iger Essigsäure gelchromatographiert.

Die mit Ninhydrin, Chlor/TDM und Wasser anfärbbare, chemisch einheitliche Fraktion (im folgenden als P1 bezeichnet) erwies sich als N-terminales Bruchstück der tryptischen Spaltung von Epidermin (siehe unten).

Der extrem hydrophobe, gallertartige Niederschlag bestand aus mehreren chemischen Komponenten, welche bei der tryptischen Spaltung aus dem C-terminalen Bruchstück des Gesamtmoleküls entstanden. Ein chemisch einheitliches Produkt (im folgenden P2 bezeichnet) erhielt man durch Auflösen des Niederschlags in Dimethylformamid und anschließender Gelchromatographie an Sephadex LH-20 (Dextrangel, durch Hydroxypropylierung aus Sephadex G-25 hergestellt, Firma Pharmacia) mit Dimethylformamid als Fließmittel. P2 ließ sich mit Wasser, Chlor/TDM und unter UV-Licht detektieren. Die Ninhydrinreaktion an P2 verlief negativ.

Die Aminosäurenanalyse des Fragments P1 ergab folgende Zusammensetzung: Pro (1), Lan (1), β-Me-Lan (1), Gly (1), Ala (2), Ile (2), Phe (1), Lys (2). Da durch Dansylierung des Gesamtmoleküls Isoleucin als N-terminale Aminosäure bestimmt wurde und das Fragment P2 kein Isoleucin enthält, muß das Fragment P1 das N-terminale Spaltprodukt des Gesamtmoleküls sein. Die C-terminale Aminosäure von Fragment P1 muß auf Grund der tryptischen Spaltung Lysin sein.

Zur weiteren Strukturaufklärung von P1 wurde die C-terminale Aminosäure Lysin enzymatisch mittels Carboxypeptidase B (Firma Boehringer Mannheim) entfernt. Das daraus entstandene Fragment P12 konnte durch Gelchromatographie an Sephadex G-25 (1% Essigsäure) in reiner Form isoliert werden. P12 besteht aus folgenden Aminosäuren: Pro (1), Lan (1), β-Me-Lan (1), Gly (1), Ala (2), Ile (2), Phe (1), Lys (1).

Die Schwefelbrücken der Thioetheraminosäuren Lanthionin und β-Methyllanthionin verhindern eine

EP 0 181 578 B1

Sequenzanalyse nach Edman. Durch Umsetzung von P12 mit Raney-Ni W2 erhielt man ein schwefelfreies Dodekapeptid. Dabei wird meso-Lan in D- und L-Alanin überführt und $\beta$-Methyllanthionin in D-Aminobuttersäure und L-Alanin. Die Dodekapeptidsequenz wurde durch Edman-Abbau und FAB-Spektrometrie aufgeklärt:

Ile[1]-Ala[2]-Ala[3]-Lys[4]-Phe[5]-Ile[6]-Ala[7]-Abu[8]-Pro[9]-Gly[10]-Ala[11]-Ala[12].

Verschiedene Untersuchungen für die Zuordnung der Schwefelbrücken beim Fragment P12 ergaben folgende Struktur:

```
              ┌──────── S ────────┐
              │                   │
   Ile-Ala-Ala-Lys-Phe-Ile-Ala-Abu-Pro-Gly-Ala-Ala
                          │                 │
                          └──── S ──────────┘
```

Das durch tryptische Spaltung erhaltene C-terminale Bruchstück P2 ließ sich wie folgt charakterisieren: Asn (1), Lan (1), Gly (1), Phe (1), Tyr (1), $\alpha$-Ketobuttersäure und S-(2-Aminovinyl)-D-cystein.

Die $\alpha$-Ketobuttersäure stammt aus der Dehydroaminobuttersäure, welche in der Sequenz des Gesamtmoleküls direkt auf Lysin[13] folgt. Die tryptische Spaltung setzt die Aminogruppe der Dehydroaminobuttersäure frei; da Dehydroaminosäuren mit freier Aminogruppe instabil sind, lagern sie sich unter anderem in $\alpha$-Ketosäuren um.

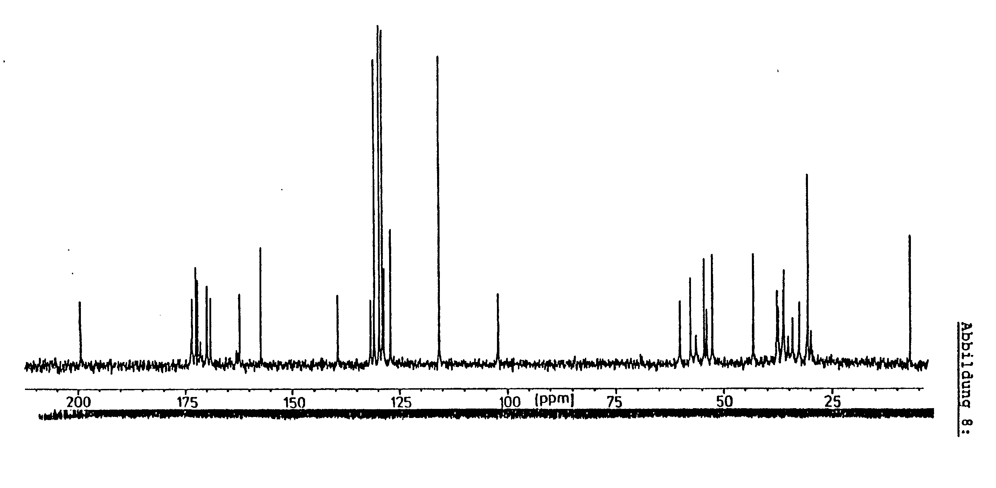

Das tryptische Fragment P2 ist N-terminal mit der $\alpha$-Ketobuttersäure blockiert (Ninhydrinreaktion an P2 verläuft negativ). Neben den durch Totalhydrolyse und Aminosäurenanalyse identifizierten Aminosäuren Lanthionin, Glycin, Phenylalanin, Tyrosin und Asparaginsäure besitzt P2 einen weiteren Baustein, der durch saure Totalhydrolyse zerstört wird. Dieser ließ sich an Hand seiner Signale in den $^{13}$C-Kernresonanzspektren von Epidermin (Abb. 6) und im Fragment P2 (Abb. 8) charakterisieren.

Einen Hinweis auf die chemische Natur dieser Aminosäure erhielt man durch Umsetzung von P2 mit Raney-Ni W2, bei der zwei Hauptprodukte entstanden. Bei beiden durch präparative HPLC isolierten Produkten zeigte sich nach Totalhydrolyse ein zusätzlicher D-Alaninrest im Aminosäurenchromatogramm, der durch diese Umsetzung entstand.

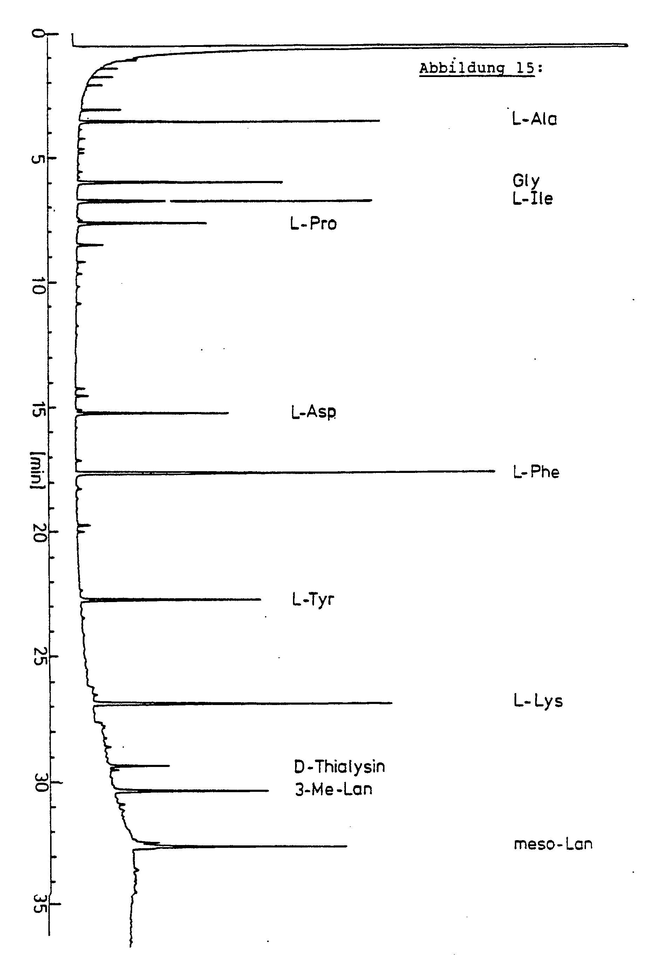

Die Struktur dieses Bausteins konnte durch heterogene Hydrierung des nativen Antibiotikums geklärt werden. Die vier Reaktionsprodukte H1, H2, H3 und H4 wurden durch semipräparative HPLC gereinigt, totalhydrolysiert und der Gaschromatographie an chiraler stationärer Phase unterworfen (Abb. 15). Dabei trat im Vergleich zum Chromatogramm in Abb. 2 bei H1 und H3 ein zusätzlicher Peak auf, der über sein Massenspektrum als S-(2-Aminoethyl)-D-cystein identifiziert wurde. In Epidermin war diese Aminosäure als säurelabiler Baustein S-(2-Aminovinyl)-D-cystein enthalten.

Die beiden aus dem C-terminalen Bruchstück P2 durch Behandlung mit Raney-Nickel W2 entstandenen Peptide P21 und P22 konnten durch Aminosäurenanalyse und Gaschromatographie an Chirasil-Val wie folgt charakterisiert werden:

| Reaktionsprodukte | P 21 | P 22 |
|---|---|---|
| Aminosäurenanalyse und Gaschromatographie an Chirasil-Val | 2 D-Ala | 1 D-Ala |
| | 1 L-Ala | meso-Lan |
| | 1 L-Phe | 1 L-Phe |
| | 1 L-Tyr | 1 L-Tyr |
| | 1 L-Asp | 1 L-Asp |
| | 1 Gly | 1 Gly |

Zusätzlich waren beide Produkte C-terminal durch Ethylamin blockiert. S-(2-Aminovinyl)-D-cystein zerfiel bei der Entschwefelung bei gleichzeitiger Hydrierung in D-Alanin und Ethylamin.

Die Sequenzierung des brückenfreien Heptapeptides P21 (Partialhydrolyse; 0.1 N-Salzsäure, 93 $^\circ$C, 16 Stunden) ergab folgende Struktur:

$H_3C-CH_2-CO-CO-Gly-D-Ala-L-Phe-L-Asn-D-Ala-L-Tyr-L-Ala-NHEt$

Die Zuordnung der in P22 enthaltenen meso-Lanthioninbrücke durch Partialhydrolyse und diverse

5

chemische Untersuchungen an enzymatischen Fragmenten aus P21 und P22 waren nur mit folgender Struktur des aus tryptischer Spaltung entstandenen Fragments P2 zu vereinbaren:

```
             ┌──────────── S ────────────┐
             │                           │
H₃C-CH₂-CO-CO-Gly-Ala-Phe-Asn-Ala-Tyr-Ala-NH-CH
                     │                       ‖
                     └────────── S ──── CH
```

Die formale Substitution der P2 N-terminal blockierenden 2-Ketobuttersäure durch die im nativen Antibiotikum vorhandene Aminosäure Dehydrobutyrin und eine anschließende Fragmentkondensation der tryptischen Peptide P1 und P2 führte direkt zu der nachstehenden Sequenz des heterodet tetracyclischen Peptidantibiotikums Epidermin:

Struktur des ribosomal synthetisierten, heterodet cyclischen Dokosapeptidantibiotikums EPIDERMIN:

```
                    meso-Lanthionin

              CH₂ ──────── S ──────── CH₂
               │                       │
  H-Ile-Ala-NH-CH-CO-Lys-Phe-Ile-NH-CH-CO─┐
               (S)                  (R)    │
                                           │
     ┌─────────────────────────────────────┘
     │
        CH₃         ß-Methyllanthionin
         │
   (S)CH ──────── S ──────── CH₂
     │   │                    │
     └ NH-CH-CO-Pro-Gly-HN-CH-CO-Ala-Lys ──┐
         (S)               (R)              │
     ┌────────────────────────────────────┘
     │
        CH₃      meso-Lanthionin
         │
        CH       CH₂──────── S ──────────── CH₂
         ‖        │              (S)         │    (Z)
     └ HN-C-CO-Gly-HN-CH-CO-Phe-Asn-HN-CH-CO-Tyr-HN-CH-CO-NH-CH
        (Z)        (S)              │              (R)        ‖
                              CH₂──── S ──────── CH
                              S-(2-Aminovinyl)-D-cystein
```

Die dem N-Terminus näherstehenden Hälften der einzelnen Thioetheraminosäuren sind jeweils D-konfiguriert (entspricht dir (S)-Konfiguration in der R,S-Nomenklatur).

Gegenstand der Erfindung ist also ein einheitliches und damit als rein anzusehendes Produkt, Epidermin genannt, desweiteren Verfahren zu seiner Herstellung, Isolierung und Reinigung.

Die Zucht des Produzenten Staphylococcus epidermidis DSM 3095 erfolgt aerobisch bei 37°C in einem Komplexmedium der Zusammensetzung 2 bis 4% Fleischextrakt, 1 bis 3% Malzextrakt und 0,25 bis 1% $CaCO_3$ oder 0,25 bis 0,5% $Ca(OH)_2$. (Bei den Prozentangaben handelt es sich hier und im folgenden immer, sofern nichts anderes angegeben wird, um Gewichtsprozente.) Das Maximum an antibiotischer Aktivität wird nach 18-23 Stunden erreicht.

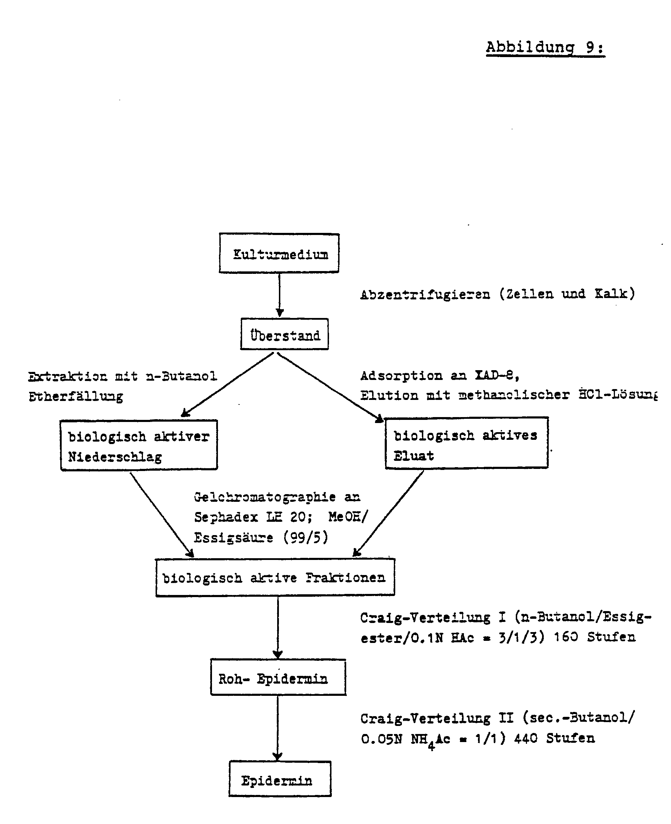

Versuche zur Anreicherung des Antibiotikums wurden beispielsweise am Kulturfiltrat von 10 l-Fermentern vorgenommen, wobei die Effektivität einzelner Schritte durch den Plattendiffusionstest überprüft wurde. Die aktive Komponente konnte durch Extraktion des von Zellen und Kalk befreiten Kulturfiltrats mit n-Butanol angereichert werden. Die Extraktion mit n-Butanol gelang aber nur beim natürlichen End-pH des Kulturfiltrats von 8,0. Eine weitere Reinigung konnte durch die Abtrennung lipidischer Begleitstoffe durch Etherfällung erreicht werden. Dazu wurde der Butanolextrakt evaporiert, der Rückstand in Methanol gelöst und in die fünffache Menge kalten Diethylether eingerührt. Die Aktivität blieb dabei vollständig im Niederschlag zurück (Schema Abb. 9).

Ein besonders geeignetes Verfahren zur Anreicherung ist auch die Adsorption des zentrifugierten Kulturfiltrats an Amberlite XAD-8 oder verwandten Typen dieses Polymeren auf Acrylesterbasis (Firma Serva). Die Anheftung von Epidermin erfolgt dabei nicht durch einfache Adsorption, sondern durch die Kationenaustauschwirkung freier Acrylsäuregruppierungen am Harz. Dafür spricht die Tatsache, daß die aktive Komponente nur durch Elution mit Methanol/konz. HCl (99:1) vom Harz gelöst werden konnte. Das stark saure Eluat muß vor dem Einengen am Vakuum mit Ammoniak neutralisiert werden. Auf ein anschließendes Umfällen aus Methanol/Ether konnte nach dieser Aufarbeitung mittels Adsorption an Amberlite XAD-8 verzichtet werden.

Eine Isolierung von Epidermin durch Adsorption kann auch bereits während der Kultivierung des Mikroorganismus direkt in der Kulturbrühe erfolgen.

Stabilitätstests und chromatographische Untersuchungen wurden am lyophylisierten Butanolextrakt durchgeführt. Die Inkubation des Extraktes mit wässrigen Lösungen verschiedener pH-Werte ergab ab pH 10 eine starke Abnahme der Aktivität, im Bereich von pH 2-7 ist das Antibiotikum jedoch stabil.

In den Dünnschichtchromatogrammen des eingeengten Butanolextraktes fanden sich eine Vielzahl mit verschiedenen Sprühreagentien anfärbbarer Verbindungen. Parallel dazu durchgeführte Bioautogramme ergaben wichtige Hinweise auf die Natur des neuen Wirkstoffs. In sauren und fast allen neutralen Systemen bleibt das Antibiotikum bei der Dünnschichtchromatographie an Kieselgel 60 am Start sitzen, während die Chromatographie mit alkalischen Laufmitteln $R_F$-Werte zwischen 0,3 und 0,75 ergab. Die Kombination der Bioautographie mit der Dünnschichtchromatographie in alkalischen Systemen zeigte eine Korrelation mit einer Verbindung, welche durch Ninhydrin anfärbbar war.

Als Ergebnis dieser Vorversuche konnte festgehalten werden, daß es sich bei dem Antibiotikum um ein stark basisches Peptid handelt, welches sich einer weiteren Aufreinigung durch Säulenchromatographie an Kieselgel 60 entzog, da es mit sauren oder neutralen Systemen nicht von der Säule eluiert werden konnte.

Die Chromatographie des Amberlite XAD-Eluats bzw. des von Lipiden befreiten Butanolextrakts an Sephadex LH-20 mit Methanol/Essigsäure (95:5) trennte eine große Zahl von kleinen Peptiden, Aminosäuren und Salzen aus dem Medium vom Antibiotikum ab (Schema Abb. 9).

Bei der sich anschließenden multiplikativen Gegenstromverteilung nach Craig konnten die bei der Extraktion des Antibiotikums aus dem Kulturfiltrat gemachten Erfahrungen herangezogen werden. In einer ersten Flüssig-Flüssig-Verteilung mit dem System n-Butanol/Essigester/0,1 N Essigsäure (3:1:3) blieb das Antibiotikum am Start sitzen. Bei der zweiten Craig-Verteilung mit dem neutralen System 2-Butanol/0,05 N Ammoniumacetat (1:1) fand man das Antibiotikum in der Mitte der Apparatur. Ammoniumacetat konnte durch Lyophilisation am Hochvakuum wieder entfernt werden.

Epidermin fiel nach Gefriertrocknung als ein in allen verwendeten Dünnschichtsystemen einheitliches Pulver an.

Während die Reinigung gemäß der Verfahrensweise der Europäischen Patentanmeldung 0 027 710 durch Gefrier-Auftau-Extraktion, Verdampfung der Extraktionsmittel, Ultrafiltration, Fällung durch Ammoniumsulfat, Ionen-Austausch-Chromatographie und Gelfiltration an Sephadex G-50 oder G-25 oder G-15 oder an Biogel P2 erfolgte, führt die erfindungsgemäße Reinigung zu einem einheitlichen Reinprodukt durch die Adsorption des zentrifugierten Kulturfiltrates an Amberlite XAD-8, gefolgt von einer Gelchromatographie an Sephadex LH-20, und anschließender zweifacher Gegenstromverteilung nach Craig. Die Isolierung und Reinigung gemäß dem Verfahren der vorstehend genannten Europäischen patentanmeldung und dem erfindungsgemäßen Verfahren erweist sich somit als völlig unterschiedlich.

Weitere Unterschiede gegenüber dem bekannten Verfahren gemäß der obengenannten EP-A-0.027.710 bestehen in der Wahl der komplexen Nährlösung. Während die bekannte Nährlösung Brain Heart Infusion (37 g BHI/l) nur eine sehr geringe Antibiotika-Ausbeute erbrachte, zeigt eine erfindungsgemäße Nährlösung, bestehend aus 2 bis 4 % Fleischextrakt, 1 bis 3 % Zucker bzw. Zuckeralkohole, wie Malzextrakt, Maltose, Galaktose, Laktose, Mannit, Glucose, Glycerin und 0,25 bis 1 % Calciumcarbonat und/oder 0,25 bis 0,5% Calciumhydroxid sehr gute Ergebnisse. Die beste Produktion wurde bei einer Nährlösung folgender Zusammensetzung erhalten: 3 % Fleischextrakt, 2 % Malzextrakt und 0,37 % Calciumhydroxid. Von allen C-Quellen zeigte Maltose nach Malzextrakt die beste Produktion. Glucose sollte nur in Verbindung mit anderen C-Quellen eingesetzt werden. Auch die Kombination von Laktose und Maltose bzw. Galaktose und Maltose lieferten gute Ergebnisse. Alle anderen üblichen C-Quellen zeigten keine oder nur eine geringe Produktion. Die Zugabe aller 20 Aminosäuren als N-Quelle in Konzentrationen von jeweils 2 g/l brachte die gleichen Ergebnisse wie der Fleischextrakt. Casaminoacid (Firma Difco) eignet sich als N-Quelle nur bei Zugabe von Tryptophan (1-2 mM) und Vitaminen. Als Vitamine eignen sich (bevorzugte Konzentrationen in Klammer): Biotin (0,006 mg/l), Nicotinsäure (2,3 mg/l), Thiamin (1,0 mg/l), Pyridoxin.HCl (12,0 mg/l), Calciumpantothenat (1,2 mg/l).

Die Fermentation erfolgt unter guter Belüftung bei Temperaturen zwischen 34 und 37°C. Den besten Produktionsverlauf erhält man, wenn der pH-Wert vor der Fermentation bei 6,0 bis 7,0 liegt. Bei Fehlen von Carbonaten oder Hydroxiden zweiwertiger Kationen, wie Calciumkarbonat oder Calciumhydroxid, findet keine Produktion statt. Nach der Zugabe von beispielsweise Calciumcarbonat zeigt der pH-Wert einen charakteristischen Verlauf mit Abfall in den sauren Bereich, dabei fand keine Produktion statt. Beim anschließendem Anstieg des pH-Wertes in den schwach alkalischen Bereich setzte die Produktion ein. Anstelle von Calciumcarbonat kann auch Magnesiumcarbonat verwendet werden, Calciumhydroxid lieferte bessere Ergebnisse als Calciumcarbonat. Mit 50 mM Calciumhydroxid konnte die Produktion gegenüber 25 mM Calciumcarbonat noch etwas gesteigert werden. Bei der Verwertung von C-Quellen (Zucker) durch den Stamm kommt es zur Bildung organischer Säuren, die durch zweiwertige Kationen komplexiert werden, wobei gleichzeitig eine Abpufferung des Mediums erfolgt.

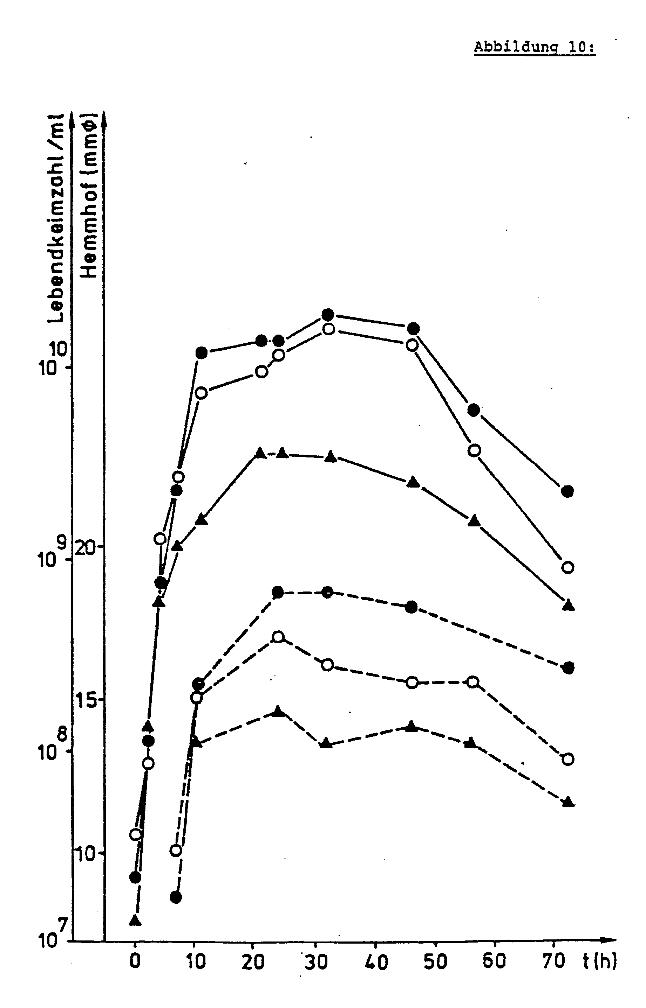

In Abbildung 10 sind die Ergebnisse anhand des Verlaufs der Lebendkeimzahl und der Antibiotikum-Produktion, ausgedrückt in mm Hemmhof gegen Micrococcus luteus ATCC 9341, der erfindungsgemäßen Medien im Vergleich zu Brain Heart Infusion Medium (Firma Difco) gemäß der oben zitierten Europäischen Patentschrift angegeben.

Mittels einer Eichgeraden ergaben sich im Plattendiffusionstest nachstehend aufgeführte Aktivitätssteigerungen zum Zeitpunkt des Produktionsmaximums.

Die Aktivität in Nährlösung Brain Heart Infusion wurde gleich 100% gesetzt.

```
a)  Brain Heart Infusion Agar                    100 %

b)  3 % Fleischextrakt, 2 % Malzextrakt,

    25 mM Calciumcarbonat                        200 %

c)  3 % Fleischextrakt, 2 % Malzextrakt,

    50 mM Calciumhydroxid                        320 %
```

Diese Angaben stellen keine absoluten Produktionswerte dar. Ein Vergleich der mit Hilfe des Plattendiffusionstests gewonnenen Werte zeigt aber eindeutig, daß gegenüber der bekannten Arbeitsweise bei Einhaltung der erfindungsgemäßen Arbeitsweise eine signifikante Steigerung der Ausbeuten erzielt wurde.

Der zur Herstellung von Epidermin verwendete Stamm wird nach Schleifer & Kloos (Int. J. Syst. Bact. 25, 50-61 (1975)) wie folgt charakterisiert:

| | |
|---|---|
| Gramfärbung : | positiv |
| Zellgröße : | Durchmesser 0,5-0,8 μm |
| Koloniengröße : | ca. 1 mm Durchmesser |
| Aussehen der Kolonien : | glatt, glänzend, in der Mitte leicht erhöht |
| Kolonienfarbe : | gräulich - weiß |
| Zellen in Kultur : | oft Einzelzellen oder Zweier-Pakete, selten größere Haufen |
| Hämolyse : | auf Blutplatten ausgestrichene Kulturen zeigten starke Hämolyse |
| Anaerobes Wachstum : | der Stamm wächst auch unter anaeroben Bedingungen |
| Lysozymempfindlichkeit: : | die Zellen sind bis 2 mg/ml resistent gegen Lysozym |
| Lysostaphinempfindlichkeit : | die Zellen sind schon bei 20 μg/ml sensitiv für Lysostaphin |
| Epidermin-Resistenz : | bis 1 mg/ml nachgewiesen |
| Sonstige Resistenzen : | in Flüssigkultur zeigt der Stamm eine ausgeprägte Resistenz gegenüber Streptomycin (bis 1 mg/ml) und Spectinomycin (0,5 mg/ml) |

Erhöhter NaCl-Gehalt : Der Stamm wächst noch gut bis 15% Gew.-% Natriumchlorid.
Kolonien oder ausgestrichene Kulturen sind sehr klebrig.

Die Verwertung von verschiedenen C-Quellen unter Säurebildung und andere Enzymreaktionen wurden mit Hilfe des Api-Staph-Systems (Firma Biomerieux, Nürtingen) bestimmt.

Dieses System beruht auf einer Kombination von 20 biochemischen Reaktionen, die auf die Klassifizierung von KLOOS & SCHLEIFER (J.Clin.Microbiol. 1, 82-88 (1975)) zurückzuführen sind.

Tabelle 1: C-Quellenverwertung und andere Enzymreaktionen

| C-Quelle bzw. Enzym | Pro-duzent | Staph. epid. Kloos&Schleifer | Staph.epid. Api - Staph |
|---|---|---|---|
| Kontrolle | - | - | - |
| D-Glucose | + | k.A. | + |
| D-Fructose | + | + | + |
| D-Mannose | (+) | (+) | + |
| Maltose | + | + | + |
| Laktose | (+) | (+) | + |
| D-Trehalose | - | - | - |
| D-Mannitol | - | - | - |
| Xylitol | - | - | - |
| D-Melibiose | - | k.A. | - |
| Raffinose | - | k.A. | - |
| D-Xylose | - | - | - |
| Saccharose | + | + | + |
| α- Methylglucosid | - | k.A. | - |
| N-Acetylglucosamin | - | k.A. | ± |
| Nitratreduktion | + | + | ± |
| Phosphatase | + | + | + |
| Bildung von Acetylmethyl-carbinol (Voges-Pros-kauer Reaktion) | + | k.A. | + |

| Arginindehydrolase | - | k.A. | $\pm$ |
| Urease | + | k.A. | + |

+    positive Reaktion

-    negative Reaktion

(+)   eindeutig positive Reaktion tritt erst etwas später auf

$\pm$    variable Eigenschaft

k.A. keine Angaben

Der Stamm Staphylococcus epidermidis DSM 3095 wurde auf Schrägröhrchen mit einem Medium der Zusammensetzung

| Pepton | 10 | g |
| Dinatriumhydrogenphosphat | 2 | g, |
| Fleischextrakt | 8 | g |
| Glucose | 10 | g (separat autoklaviert) |
| Kochsalz | 3 | g |
| pH | 7,2 | |

überimpft, über Nacht bei 37° C inkubiert und anschließend bei -20° C eingefroren. Für neue Versuchsansätze wurde jeweils ein frisches Röhrchen aufgetaut, da bei längerer Lagerung bei 4° C Aktivitätsverluste beobachtet wurden.

Im folgenden wird eine genauere Beschreibung zur Herstellung des Antibiotikums Epidermin gegeben: Die Fermentation läßt sich in dazu geeigneten Schüttelkolben durchführen, zur Herstellung größerer Substanzmengen können auch Fermenter von 200 Liter und mehr verwendet werden.

Für Kolbenversuche wurden 500 ml Erlenmeyerkolben mit einem seitlichen Einstich verwendet. Die Kolben wurden mit 100 ml Nährlösung gefüllt und 20 Minuten bei 121° C autoklaviert. Als Impfmaterial diente 1% einer 4 Stunden alten Vorkultur. Die Inkubation erfolgte bei 37° C auf der rotierenden Schüttelmaschine bei 140 rpm.

Für die Fermentation im 10-Liter-Maßstab wurden Fermenter mit 10 l Nutzinhalt (Modell MF-14 New Brunswick, Scientific Co., New Brunswick, USA) mit 9,9 l Nährlösung gefüllt und 30 Minuten bei 134° C autoklaviert. Nach dem Abkühlen wurde mit 100 ml einer 4 Stunden alten Vorkultur beimpft und bei 37° C, 0,6 vvm und 240 rpm des Blattrührers fermentiert. Der relativ starken Schaumbildung wurde durch wiederholte Zugabe von sterilem Polyol begegnet.

Für die Fermentation im 25 l Maßstab wurde ein 25 l Fermenter (Typ b 25, Braun/Melsungen mit Umwurfsystem) mit 25 l Nährlösung unter Zusatz von 3 ml Polyol beschichtet und in situ bei 121° C 30 Minuten sterilisiert. Als Impfmaterial dienten 300 ml einer 4 Stunden alten Vorkultur. Die Fermentation erfolgte bei 37° C, 0,6 vvm und 1000 rpm.

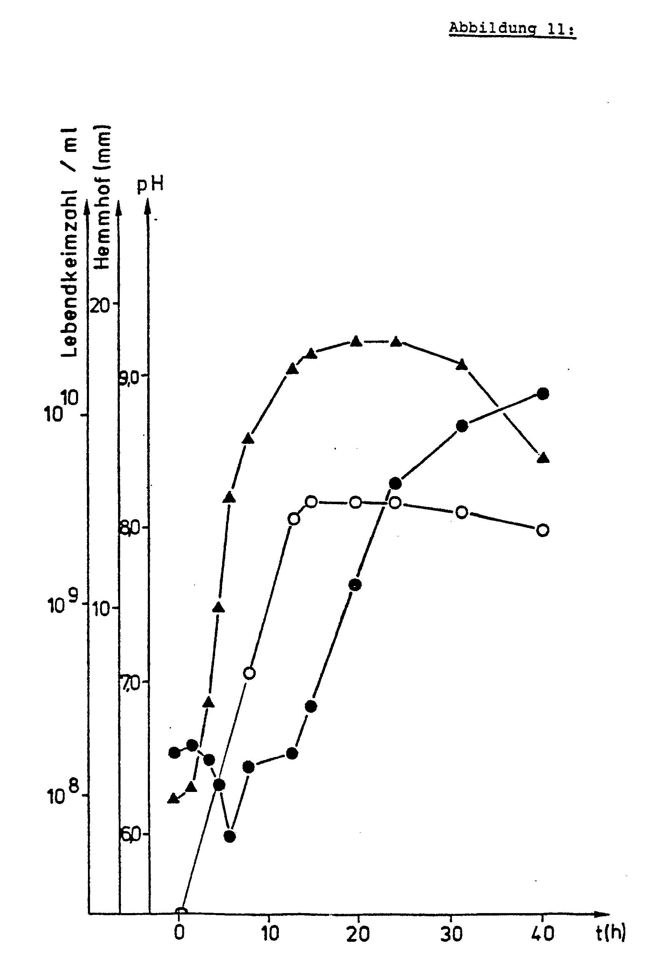

Der Verlauf einer Fermentation im 10 l Maßstab in einer Nährlösung, enthaltend 30 g Fleischextrakt, 20 g Malzextrakt und 5 g Calciumcarbonat in 1 Liter, ist in Abbildung 11 dargestellt. Intensives Wachstum verbunden mit der Verwertung der angebotenen C-Quelle unter Säurebildung kann am Abfall des pH-Wertes erkannt werden. Mit Beginn der Alkalisierung kann das Antibiotikum im Kulturfiltrat nachgewiesen werden. Die Produktion erreicht nach 12 bis 18 Stunden ihr Maximum.

Zur Kontrolle des Fermentationsverlaufes wurden zu verschiedenen Zeiten während der Fermentation steril Proben entnommen.Die Proben wurden folgendermaßen ausgewertet:

a.) pH-Wert:

Messung mit einem Labor-pH-Meter (Knick pH-mV-Meter)

b) Wachstumsverlauf:

Das Wachstum konnte anhand der Zunahme der Lebendkeimzahl verfolgt werden.

Hierzu wurden 0,5 ml steril entnommene Kultur in Saline verdünnt und davon 0,1 ml auf Platten (Medium: Pepton 10 g, Fleischextrakt 8 g, Kochsalz 3 g, Dinatriumhydrogenphosphat 2 g, Glucose 10 g auf 1 l.) ausplattiert. Nach 18 Stunden Inkubation bei 37°C konnten die Einzelkolonien ausgezählt werden.

c.) Antibiotikumkonzentration:

Die Proben wurden in einer Eppendorf-Zentrifuge 3200 2 Minuten abzentrifugiert und 20 $\mu$l des Überstandes im Plattendiffusionstest getestet.

Parallel wurde eine Eichkurve mit bekannten Konzentrationen erstellt.

Nach Erreichen des Produktionsmaximums wurde die Kulturflüssigkeit mittels kontinuierlicher Zentrifugation (Zentrifuge: Typ LA 71b-4, Loher & Söhne, Ruhstorf/Rott) mit 1380 rpm abzentrifugiert. Für eine optimale Abtrennung der Zellen mußte die Durchflußrate sehr niedrig gehalten werden. Eine erste Anreicherung der aktiven Komponenten wurde mittels der auf Seite 10 erwähnten Adsorption an Amberlite XAD-8, gemäß nachfolgendem Beispiel 1 erreicht:

Beispiel 1

80 Liter Kulturfiltrat wurden über ca. 8 Liter Amberlite XAD-8 gegeben. Im Durchlauf konnte keine Aktivität festgestellt werden. Beim anschließenden Waschen mit Wasser wurde ebenfalls keine Aktivität eluiert. Anschließend wurde mit 13 Liter Methanol gewaschen. Im Waschwasser wurde keine Aktivität detektiert. Die Elution erfolgte mit Methanol/Salzsäure 99:1, wobei folgende Fraktionen erhalten wurden:

```
Fraktion 1:    0,8 l aktiv
Fraktion 2:    4,5 l Hauptaktivität
Fraktion 3:    2   l aktiv
Fraktion 4:    2   l keine Aktivität
Fraktion 5:    1   l keine Aktivität
```

Die Fraktionen wurden dünnschichtchromatographisch auf ihren Gehalt an Epidermin getestet, anschließend wurden die aktiven Fraktionen vereinigt und am Rotationsverdampfer zur Trockne eingeengt (81,2 g). Der Rückstand wurde in Methanol/Essigsäure (95/5) aufgenommen (400 ml), von unlöslichen Bestandteilen abzentrifugiert und in Portionen von je 50 ml an Sephadex LH-20 (Säule 100x5 cm, Fließmittel Methanol/Essigsäure 95:5) gelchromatographiert. Positive Fraktionen wurden vereinigt und eingedampft (14,8 g).

Für die multiplikative Verteilung nach Craig kamen Apparaturen der Firma Labortec (Basel) zum Einsatz. Eine erste Trennung wurde mit einer 50 ml-Apparatur im System n-Butanol/Essigester/0,1 M Essigsäure (3/1/3) durchgeführt. Die in der Unterphase (100 ml) gelöste Probe (5 g) wurde folgenden Trennbedingungen unterworfen:

Stufenzahl:                160
Schüttelbewegungen/Stufe:  70
Schüttelintensität:        45
Trennzeit:                 20 min

Zur Endreinigung wurde das erhaltene Rohprodukt (4,2 g) in Portionen von je 2 g in der Unterphase des Systems 2-Butanol/0,05 N Ammoniumacetat (50 ml) gelöst und in einer 10 ml-Apparatur mit 440 Elementen aufgereinigt.

Stufenzahl:                440
Schüttelbewegungen/Stufe:  50
Schüttelintensität:        50
Trennzeit:                 10 min

Die Epidermin enthaltenden Elemente wurden vereinigt, eingeengt, mit Wasser aufgenommen und mehrmals am Hochvakuum lyophylisiert. Das Antibiotikum (2,6 g) erwies sich bei allen vorgenommenen Untersuchungen als einheitlich.

Während der Herstellung und Isolierung des Epidermins wurde zur biologischen Charakterisierung der Plattendiffusionstest angewandt; nämlich zur Aufnahme des Wirkungsspektrums sowie zur Kontrolle von Produktion, Aufarbeitung und Anreicherung. Der Test wurde, wie bei Zähner und Maas, Biology of

11

antibiotics (Springer Verlag, Berlin-Heidelberg-New York, 1972) beschrieben, in Petrischalen der Firma Greiner, Nürtingen mit Filterrondellen von 6 mm Durchmesser (Macherey & Nagel, Düren) durchgeführt. Die Filterrondellen wurden mit 20 μl der betreffenden Testflüssigkeit benetzt, bei Raumtemperatur auf einer Glasplatte getrocknet und auf die Testplatten aufgelegt. Die Testplatten wurden bei 37°C inkubiert und nach ca. 16 Stunden die Wachstumshemmung ausgewertet.

Als Routinetestkeim diente Streptococcus pyogenes ATCC 8668 und, aufgrund seiner problemloseren Handhabung, Micrococcus luteus ATCC 9341.

Testplatten mit Streptococcus pyogenes ATCC 8668:

Der Testkeim mußte vor Herstellung der Testplatten auf Blutplatten mit Mucin [Medium: 500 ml Agar pH 7,4, 160 ml Mucinlösung (10 Gewichtsprozent), 3,5 ml Glucoselösung (50 Gewichtsprozent), 70 ml Hammelblut] frisch überimpft werden. Nach 15-18 Stunden Inkubation bei 37°C wurde eine Salinesuspension (E$_{578}$ 0,5) hergestellt und 1 ml dieser Suspension zu 100 ml Nährboden (Medium: Pepton 10 g, Fleischextrakt 8 g, Kochsalz 3 g, Na$_2$HPO$_4$ 2 g, Glucose 10 g auf 1 l, pH 7,2) pipettiert. Die zu 10 ml Portionen gegossenen Platten waren einige Tage bei 4°C haltbar.

Testplatten mit Micrococcus luteus ATCC 9341:

Eine Übernachtkultur (Medium: Pepton 10 g, Fleischextrakt 8 g, Kochsalz 3 g, Na$_2$HPO$_4$ 2 g, Glucose 10 g auf 1 l, pH 7,2) wurde auf eine Extinktion von 1,0 bei 578 nm verdünnt. Mit 0,25 ml dieser Suspension wurden 100 ml Agar angeimpft und Platten zu 10 ml gegossen. Die Testplatten konnten mehrere Tage bei 4°C aufbewahrt werden.

Testpatten für das Wirkungsspektrum:

Testplatten mit Bakterien:

α.) Aerob lebende Bakterien:

Übernachtkulturen wurden in den jeweiligen Testmedien angezogen und die Platten wie bei Micrococcus luteus ATCC 9341 beschrieben hergestellt.

β.) Anaerob lebende Bakterien:

Die Vorkulturen für Clostridium pasteurianum ATCC 6013 und Propionibacterium acnes DSM 1897 wurden in Reagenzgläsern gezogen, die zur Verdrängung des Sauerstoffes bis zum Wattestopfen mit Nährlösung aufgefüllt wurden. Mit 3 ml Vorkultur wurden 100 ml Agar beimpft und Platten in 10 ml Portionen gegossen.

Die Inkubation erfolgte im Anaerobier-Topf (BBL-Gas Pak 100, Becton, Dickinson GmbH, Heidelberg) bei der jeweils optimalen Temperatur.

Testplatten mit hefeartig wachsenden Pilzen:

In Schüttelkultur wurden die Zellen im jeweiligen Testmedium 18-20 Stunden angezogen. Nach Auszählen in der Thoma-Zählkammer wurden die Testplatten zu einer Dichte von 10$^6$ Organismen/ml Agar angeimpft.

Testplatten mit Pilzen und Streptomyceten:

Die Testorganismen wurden auf Schrägröhrchen (Medium: Hefeextrakt 4 g, Malzextrakt 10 g, Glucose 4 g auf 1 l) bei den entsprechenden Temperaturen bis zur Sporulation angezogen. Die Sporen wurden mit 3 ml Saline-Tween 80 (1 Tropfen Tween 80 auf 100 ml Saline) abgeschwemmt und in Testplatten eingegossen.

Die Substanz Epidermin besitzt eine sehr gute antibakterielle Wirksamkeit; sie wirkt besonders gut gegen eine ganze Reihe grampositiver Bakterien. Die antibakterielle Wirkung von Epidermin wurde vergleichend mit der von Nisin (Nisin vgl. u.a. DE-A-2 000 818 bzw. GB-B-1 182 156) untersucht; bei einigen wichtigen, klinisch relevanten Keimen wurde auch Fusidinsäure in den Vergleich miteinbezogen. Die Wirksamkeit wurde mit Hilfe des Plattendiffusionstests und durch die Bestimmung der minimalen Hemmkonzentrationen ermittelt.

a.) Plattendiffusionstest:

Im Plattendiffusionstest wurde die Empfindlichkeit verschiedener Mikroorganismen gegen Epidermin und Nisin getestet. Tabelle 2 zeigt, daß beide Antibiotika fast ausschließlich auf grampositive Bakterien wirken. Verwendet wurde Nisin mit einer Aktivität von 40000 units.

Tabelle 2: Empfindlichkeit gegen Epidermin und Nisin
Angabe in mm Hemmhof, Konzentrationen 1 mg/ml und
0,5 mg/ml.

| Mikroorganismen | Kulturbed. | | Epidermin | | Nisin | |
|---|---|---|---|---|---|---|
| | Temp. | Medium | 1 | 0,5 | 1 | 0,5 |

Bakterien
Eubacteriales, grampositiv
----------------------------

| Mikroorganismen | Temp. | Medium | Epidermin 1 | Epidermin 0,5 | Nisin 1 | Nisin 0,5 |
|---|---|---|---|---|---|---|
| Arthrobacter aurescens | 27°C | 5 | 8 | Sp | 9 | 8 |
| Arthrobacter crystallopoietes | 27°C | 5 | 16 | 15 | 16 | 15 |
| Arthrobacter globiformis | 27°C | 5 | 14 | 12 | 14 | 13 |
| Arthrobacter oxydans | 27°C | 5 | 12 | 11 | 11 | 10 |
| Arthrobacter pascens | 27°C | 5 | 13 | 12 | 14 | 13 |
| Bacillus cereus | 37°C | 3 | 8 | Sp | - | - |
| Bacillus pumilus | 37°C | 4 | 14 | 12 | 12 | 10 |
| Bacillus subtilis ATCC 6051 | 37°C | 4 | 12 | 11 | 9 | 8 |
| Bacillus subtilis ATCC 6051 | 37°C | 6 | 17 | 16 | 14 | 12 |
| Bacillus subtilis ATCC 6633 | 37°C | 4 | 14 | 11 | 9 | 8 |
| Bacillus subtilis F 24-2 | 37°C | 4 | 13 | 11 | 8 | Sp |
| Bacillus subtilis A 14 | 37°C | 4 | 12 | 10 | 9 | Sp |
| Brevibacterium flavum | 37°C | 4 | 11 | 10 | 8 | 7 |
| Clostridium pasteurianum | 30°C | 7 | 17 | 15 | 14 | 13 |
| Clostridium sporogenes | 37°C | 8 | 10 | 9 | 8 | Sp |
| Corynebacterium spec. | 27°C | 4 | 15 | 13 | 9 | 8 |
| Corynebacterium insidiosum | 27°C | 4 | 19 | 17 | 10 | 8 |
| Corynebacterium rathayi | 27°C | 4 | 14 | 12 | 10 | 9 |
| Micrococcus luteus ATCC 381 | 27°C | 5 | 10 | 9 | - | - |
| Micrococcus luteus ATCC 9341 | 27°C | 2 | 21 | 20 | 17 | 16 |
| Propionibacterium acnes | 37°C | 8 | 22 | 19 | 21 | 18 |
| Sarcina lutea | 37°C | 5 | 15 | 14 | 15 | 14 |
| Staphylococcus aureus Tü 202 | 37°C | 4 | 13 | 10 | 10 | 9 |

| Staphylococcus aureus DSM 683 | 37°C | 4 | 11 | 9 | Sp | - |
| Staphylococcus aureus Pen.res | 37°C | 4 | 12 | 11 | 9 | 8 |
| Staphylococcus cohnii | 37°C | 2 | 14 | 12 | 9 | - |
| Streptococcus pyogenes | 37°C | 2 | 22 | 20 | 18 | 17 |

Eubacteriales, gramnegativ:
----------------------------

| Proteus mirabilis | 37°C | 4 | 11 | 9 | 9 | 7 |
| Proteus vulgaris | 37°C | 5 | 9 | 8 | 8 | Sp |

Actinomycetales:
-----------------

| Streptomyces glaucescens | 27°C | 3 | 10 | 8 | 10 | 9 |
| Streptomyces violaceoruber | 37°C | 3 | 9 | 8 | - | - |
| Streptomyces virido-chromogenes | 37°C | 3 | 11 | 10 | 9 | 8 |

(-) bedeutet, daß keine Wirkung eintrat;

Sp = Spuren

b.) Minimale Hemmkonzentration:

Die minimale Hemmkonzentration für klinisch relevante Keime, angegeben in $\mu$g/ml, wurde in Mikrotiterplatten in folgendem Medium geprüft:
5 ml Na-Lactat, 5 g $Na_2SO_4$, 0,5 g $KH_2PO_4$, 0,1 g $MgCl_2$, 5 g $NH_4Cl$, 10 g Glucose, 50 $\mu$g Calciumpantothenat, 50 $\mu$g Thiamin, 0,25 $\mu$g Folsäure, 50 $\mu$g Niacin, 25 $\mu$g p-Aminobenzoesäure, 50 $\mu$g Pyridoxin-Hydrochlorid, 25 $\mu$g Riboflavin in 1000 ml Aqua dest.
In Tabelle 3 sind die minimalen Hemmkonzentrationen ($\mu$g/ml) von Epidermin, Nisin und Fusidinsäure im Vergleich dargestellt. Das hierbei verwendete Nisin besaß eine Aktivität von 2500 units.

Tabelle 3:

Minimale Hemmkonzentration in µg/ml:

| | Epidermin | Fusidinsäure | Nisin |
|---|---|---|---|
| St. epidermidis WG 99 | 2 | 4 | 128 |
| Sc. pyogenes ATCC 8668 | 0,125 | 1 | 4 |
| Sc. pneumoniae ATCC 6302 | 2 | 16 | 32 |
| Mc. luteus ATCC 15957 | 0,25 | 0,5 | 16 |
| Mc. luteus ATCC 9341 | $\leq$ 0,06 | 0,5 | 8 |
| Cb. xerosis NCTC 9755 | 0,125 | 0,125 | 16 |
| E. coli ATCC 11775 | 128 | >128 | >128 |
| E. coli ATCC 9637 | 128 | >128 | >128 |
| Propionib. acnes PC 904 | 0,06 | 0,5 | 32 |
| Propionib. acnes ATCC 25746 | 0,25 | 1 | 64 |

Die in der Tabelle 2 genannten Medien werden nachfolgend beschrieben. Die Medien wurden nach Einstellen des pH-Wertes 20 Minuten bei 121°C autoklaviert.

Die Mengenangaben beziehen sich jeweils auf 1 l Wasser. Bei chemisch definierten Medien wurde entionisiertes Wasser verwendet.

Medium:

      (2)   Glucose-Agar-Medium:

           Pepton          10 g

           Fleischextrakt    8 g

           NaCl            3 g

| | | |
|---|---|---|
| Na$_2$HPO$_4$ | 2 g | |
| Glucose | 10 g | (getrennt autoklaviert) |
| Agar | 20 g | |
| pH 7,2 | | |

(3)  Hefe-Malz-Medium:

| | |
|---|---|
| Hefeextrakt | 4 g |
| Malzextrakt | 10 g |
| Glucose | 4 g |
| Agar | 20 g |
| pH 7,3 | |

(4)  Oxoid-Medium für Bakterien:

| | |
|---|---|
| Fleischextrakt | 10 g |
| Pepton | 10 g |
| NaCl | 5 g |
| Agar | 20 g |
| pH 7,2 | |

(5)  Nutrient broth          8 g

(Firma Difco)

| | |
|---|---|
| Agar | 20 g |
| pH 7,2 | |

(6)  Minimalmedium für Bakterien (HÜTTER et al., 1966):

| | | |
|---|---|---|
| D-Glucose | 8 | g |
| di-Ammoniumtartrat | 4 | g |
| NaCl | 5 | g |
| K$_2$HPO$_4$ | 2 | g |
| MgSO$_4$ x 7 H$_2$O | 1 | g |
| CaCl$_2$ | 0,2 | g |
| MnSO$_4$ x H$_2$O | 0,01 | g |
| Ferrioxamin B | 0,02 | g |
| Agar | 20 | g |
| pH 7,2 | | |

(7)  Medium für Clostridium pasteurianum:

| | | |
|---|---|---|
| Fleischextrakt | 3 | g |
| Hefe Extrakt | 3 | g |
| Malz Extrakt | 3 | g |
| Pepton | 20 | g |
| D-Glucose | 5 | g |
| Ascorbinsäure | 0,2 | g |
| Agar | 20 | g |
| pH 7,0 | | |

(8)  Brewer Thioglycolatmedium für Propionibacterium acnes:

| | | |
|---|---|---|
| Thioglycolatmedium | 40,5 | g |
| Agar | 20 | g |
| pH  7,2 | | |

Epidermin ist sehr gut verträglich, bei topischer Anwendung wurden keine toxischen Wirkungen festgestellt.

Von sehr großem Vorteil ist es, daß die resistente Mutante Staphylococcus epidermidis DSM 3095 gegenüber dem von ihm produzierten Epidermin resistent ist. Der Stamm NCIB 11536 besitzt keine derartige Resistenz gegen das von ihm produzierte niedermolekulare Antibiotikum.

Der neue Klon DSM 3095 wurde wie folgt gewonnen:

Die Adaption wurde in 100 ml Erlenmeyerkölbchen mit einem seitlichen Einstich und 10 ml Nährlösung (Brain Heart Infusion) durchgeführt. Die Startkultur wurde mit 0,5 ml einer 12 Stunden alten Kultur angeimpft. Für die Kölbchen, die steigende Epidermin-Konzentrationen enthielten, dienten 0,5 ml der jeweils vorangegangenen, gutgewachsenen Kultur als Impfmaterial. Die Beurteilung des Wachstums erfolgte photometrisch bei 578 nm. Die Kultur, die bei einer Konzentration von 1,0 mg/ml Epidermin in Flüssigkultur noch gut gewachsen war, wurde in Saline verdünnt und auf einer Platte, in die 0,5 mg/ml Epidermin eingegossen waren, ausplattiert. Als Vergleichswert wurde eine unverdünnte 12 Stunden alte Kultur des literaturbekannten Vergleichsstammes NCIB 11536 ausplattiert. Nach 24 Stunden Inkubation bei 37° C zeigten sich bei der auf $10^{-6}$ verdünnten adaptierten Kultur 94 Einzelkolonien. Der unverdünnt auf 0,15 mg/ml Epidermin enthaltende Platten ausplattierte Vergleichsstamm wuchs auch nach 48 Stunden Inkubation nicht. Die resistenten Kolonien wurden selektioniert und auf Platten Medium 2 im Raster ausgestrichen. Nach einem Vortest auf Produktion durch Ausstechen von Stückchen mit 5 mm Durchmesser und Aktivitätstest auf Micrococcus luteus ATCC 9341 wurden produzierende Klone im Flüssigmedium getestet.

Vergleich des resistenten Stammes mit dem literaturbekannten Stamm:

a.) Minimale Hemmkonzentration im Plattendiffusionstest:

Auf Testplatten mit den beiden Stämmen ergaben sich die in Tabelle 4 aufgeführten minimalen Hemmkonzentrationen.

### Tabelle 4: Minimale Hemmkonzentrationen von Epidermin im Plattendiffusionstest bei NCIB 11536 und DSM 3095.

| Stamm | µg/ml |
|-------|-------|
| NCIB 11536 | 10 |
| DSM 3095 | > 1000 |

b.) Wachstum und Produktion:

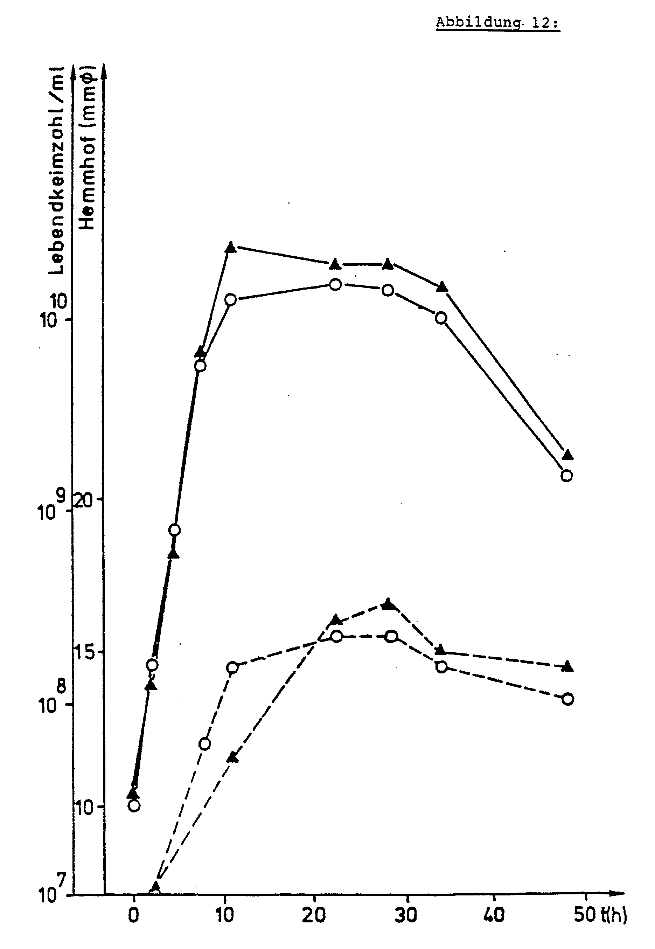

Wachstum- und Produktionsverlauf der beiden Stämme wurden in Nährlösung (3% Fleischextrakt, 2% Malzextrakt, 0,37% Ca(OH)$_2$) verglichen. Siehe Abbildung 12.

Wachstumsverlauf:     Lebendkeimzahlbestimmung

Produktionsverlauf:     Aktivität gegen Micrococcus luteus ATCC 9341 im Plattendiffusionstest.

Beim resistenten Stamm wird eine bessere Produktion beobachtet.

c.) Empfindlichkeit gegen Epidermin in verschiedenen Wachstumsphasen:

Die Versuche zur Eigenhemmung des Produzentenstammes durch das Antibiotikum und die Untersuchung auf Resistenz der selektionierten Klone wurden im Biophotometer (Eppendorf-Photometer mit Umlaufautomat) durchgeführt.

Diese Versuche können z.B. im einzelnen wie folgt durchgeführt werden:

Jeweils 0,5 ml einer 12 Stunden Kultur von Staphylococcus epidermidis NCIB 11536 bzw. des selektionierten resistenten Stammes DMS 3095 wurden auf frisches Medium überimpft.

Diese Kulturen ließ man bis zu einer Extinktion von 0,043 bei 578 nm hochwachsen (Küvetten mit einer Schichtdicke von 1 cm) und verteilte sie dann in Portionen zu 7,5 ml in die Küvetten des Biophotometers (Schichtdicke 2 cm).

Die Dichte der Zellsuspension wurde im Biophotometer auf eine Transmission von 90% eingestellt. Die Inkubation erfolgte bei 37° C und maximaler Belüftung. Epidermin wurde als wässrige Lösung in unterschiedlichen Wachstumsphasen zugegeben.

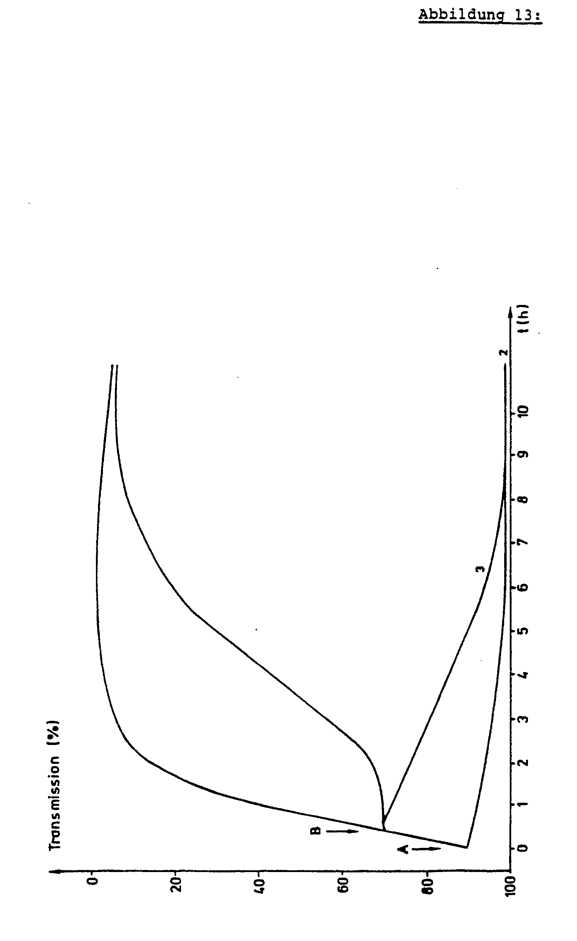

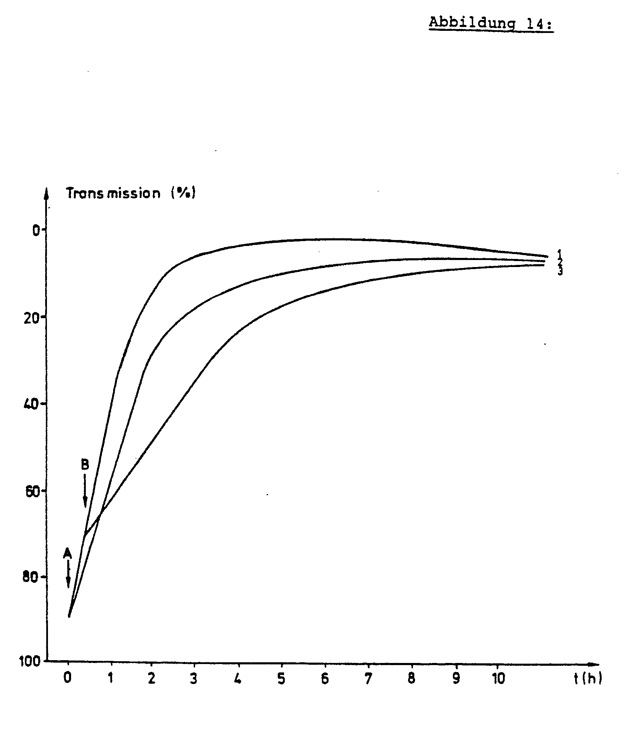

Bei beiden Stämmen wurden zu Beginn und in der Mitte der logarithmischen Phase unterschiedliche Epidermin-Konzentrationen zugegeben. Die Ergebnisse sind in Abbildung 13 und 14 dargestellt.

Mit den bis jetzt getesteten Konzentrationen konnte beim resistenten Stamm keine Lyse beobachtet werden.

Das erfindungsgemäße Antibiotikum Epidermin übt, genauso wie die Kulturbrühe, aus der es isoliert wurde, eine bakterizide Wirkung aus, die zur Lysis der Zellen führt.

Angesichts der bakteriziden Wirkung und des breiten Wirkungsspektrums gegen grampositive Bakterien ist das erfindungsgemäße Polypeptid-Antibiotikum Epidermin besonders wirksam bei der Behandlung von Infektionen, die durch grampositive Bakterien verursacht wurden. Epidermin ist besonders wertvoll für die Bekämpfung von Hautinfektionen, wie Ekzeme, Impetigo, Cellulitis und insbesonders Akne. Die sehr gute Wirksamkeit gegen einige wichtige Propionibakteriumacnes-Stämme wurde in den Tabellen 2 und 3 gezeigt. Es kann gesagt werden, daß Epidermin, welches normalerweise von Organismen, die die menschliche Haut besiedeln, erzeugt wird, bessere Aktivitäten zum Schutze der Haut ausübt als sonstige übliche Antibiotika.

Die eingangs erwähnten pharmazeutischen Zubereitungsformen, die das erfindungsgemäße Antibiotikum zusammen mit pharmazeutisch üblichen Hilfsstoffen und Trägerstoffen enthalten, können der oralen, parenteralen, enteralen oder topischen Applikation dienen. Solche Zubereitungsformen, insbesondere aber topische Zubereitungsformen können als Lösungen, Emulsionen, Gele, Lotionen, Salben, Cremes oder Puder vorliegen.

EP 0 181 578 B1

Die folgenden Beispiele zeigen die Herstellung solcher pharmazeutischer Anwendungsformen:

Beispiel 2

Tinktur

In 100 g Tinktur sind enthalten:

```
Epidermin                      1,0 g
Ethanol (94,5 Vol.-%)         56,0 g
1,2-Propylenglykol            40,0 g
Wasser demineralisiert         3,0 g
```

Herstellung:
Epidermin wird in dem Gemisch Ethanol/1,2-Propylenglykol/Wasser gelöst, die Lösung anschließend sterilfiltriert.

Beispiel 3

Lotio

In 100 g Lotio sind enthalten:

```
Epidermin                                      1,00 g
1,2-Propylenglykol                             7,00 g
Alkyldimethylbenzylammonium-
chlorid (Benzalkon A(R))                        0,15 g
Sorbitanmonopalmitat (Span 40(R))              0,40 g
Sorbimacrogolpalmitat (Tween 40(R))            1,20 g
Ölsäuredecylester (Cetiol V(R))                2,40 g
Gemisch aus Cetyl- und Stearyl-
alkohol (Lanette O(R))                          1,60 g
Cetylpalmitat                                   0,80 g
demineralisiertes Wasser      ad      100      g
```

Herstellung:
Die vorgenannten Mengen an Alkyldimethylbenzylammoniumchlorid, Sorbitanmonopalmitat, Sorbimacrogolpalmitat, Ölsäuredecylester, Cetyl- und Stearylalkohol und Cetylpalmitat werden in 75 ml Wasser eingerührt, die gefilterte Lösung von Epidermin in 1,2-Propylenglykol und dem restlichen Wasser wird dazugerührt und die Tinktur homogenisiert.

Beispiel 4

Gel

100 g Gel enthalten

| Epidermin | | 1,0 g |
|---|---|---|
| Polyethylenglykolether des Laurylalkohols (Brij 35[(R)]) | | 1,0 g |
| 1,2-Propylenglykol | | 5,0 g |
| Acrylsäurepolymerisat (Carbopol 934[(R)]) | | 1,2 g |
| p-Hydroxybenzoesäuremethylester | | 1,6 g |
| p-Hydroxybenzoesäurepropylester | | 0,4 g |
| Parfüm | | q.s. |
| Natronlauge | ad | pH 6,5 |
| demineralisiertes Wasser | ad | 100 g |

Herstellung:

Die vorgegebenen Mengen an Hilfsmitteln werden in 75 ml Wasser eingerührt; das Epidermin wird in dem Gemisch aus 1,2-Propylenglykol und dem restlichen Wasser gelöst und diese Lösung ebenfalls eingerührt; das fertige Gel wird nochmals homogenisiert.

Legenden zu den Abbildungen:

Abb. 1: Aminosäurenchromatogramm des sauren Totalhydrolysats von Epidermin; Ninhydrin-Färbung, $\lambda$ = 570 nm.

Abb. 2: Gaschromatogramm der N-Pentafluoropropionyl-aminosäure-n-propylester des sauren Totalhydrolysats von Epidermin an Chirasil-Val. Temperaturprogramm 3 Min. 85° C isotherm, dann 4° C/Min. bis 200° C; Trägergas $H_2$ (0,92 bar).

Abb. 3: UV-Spektrum von Epidermin in Wasser, pH 3 (C = 0,15 mg/ml).

Abb. 4: Infrarot-Spektrum von Epidermin in einer Kaliumbromidtablette.

Abb. 5: $^1$H-Kernresonanzspektrum von Epidermin (20 mg/0,5 ml $D_7$-Dimethylformamid, 400,16 MHz).

Abb. 6: $^{13}$C-Kernresonanzspektrum von Epidermin (40 mg/0,5 ml $^{12}$C, $^2$H-Dimethylformamid, 100,6 MHz, 45360 Pulse).

Abb. 7: HPLC-Chromatogramm von Epidermin an $\mu$ Bondapak $C_{18}$ (300x3,9 mm). Mobile Phase: A = Acetonitril/0,01 M $KH_2PO_4$ (10/90), B = Acetonitril/0,01 H $KH_2PO_4$ (70/30); linearer Gradient von 10% B auf 100% B in 30 Min., Fließgeschwindigkeit 2 ml/min.

Abb. 8: $^{13}$C-Kernresonanzspektrum des Fragments P2 (12 mg/0,5 ml $^{12}$C, $^2$H-Dimethylformamid, 100,6 MHz, 38300 Pulse).

Abb. 9: Isolierung von Epidermin.

Abb. 10: Vergleich der Medien anhand Lebendkeimzahl (durchgezogene Linien) und Aktivität gegen Micrococcus luteus (gestrichelte Linien)
▲ Brain Heart Infusion
○ 3% Fleischextrakt 2% Malzextrakt 0,25% $CaCO_3$
● 3% Fleischextrakt 2% Malzextrakt 0,37% $Ca(OH)_2$

Abb. 11: Verlauf einer Fermentation im 10 1-Maßstab
● pH-Verlauf
▲ Lebendkeimzahl
○ Aktivität gegen Streptococcus pyogenes ATCC 8668

Abb. 12: Vergleich von literaturbekanntem Stamm und resistentem Klon DSM 3095 anhand Lebendkeimzahl (durchgezogene Linien) und Aktivität gegen Micrococcus luteus ATCC 9341 (gestrichelte Linien).
○ literaturbekannter Stamm
▲ resistenter Klon

Abb. 13: Epiderminzugabe in verschiedenen Wachstumsphasen bei Staphylococcus epidermidis NCIB 11536:
Kurve 1: normaler Wachstumsverlauf

Kurve 2: Zugabe von 10 μg/ml Epidermin zu Beginn der logarithmischen Phase führt zur Lyse.

Kurve 3: Zugabe von 10 μg/ml Epidermin in der Mitte der logarithmischen Phase führt ebenfalls zur Lyse.

Kurve 4: Zugabe von 2,5 μg/ml Epidermin in der Mitte der logarithmischen Phase führt zur Wachstumsverzögerung.

Abb. 14: Epiderminzugabe in verschiedenen Wachstumsphasen beim resistenten Klon DSM 3095

Kurve 1: normaler Wachstumsverlauf, identisch mit dem Stamm NCIB 11536.

Kurve 2: Zugabe von 120 μg/ml Epidermin zu Beginn der logarithmischen Phase ergibt nur eine geringfügige Wachstumsverzögerung .

Kurve 3: Zugabe von 360 μg/ml Epidermin in der Mitte der logarithmischen Phase führt auch hier nur zu einer Wachstumsverzögerung.

Abb. 15: Gaschromatogramm des N-Pentafluoropropionyl-aminosäure-n-propylester eines Totalhydrolysats von H1 an Chirasil-Val. Temperaturprogramm 3 Min. 80°C isotherm, dann 4°C/Min. bis 200°C, Trägergas $H_2$.

**Patentansprüche**

1. Antibiotisch wirksames Polypeptid, genannt Epidermin, gekennzeichnet durch folgende Aminosäurenzusammensetzung: Asn (1), Pro (1), Gly (2), Ala (2), Ile (2), Phe (2), Lys (2), Lan (2), β-Me-Lan (1), Dhb (1), Tyr (1), S-(2-Aminovinyl)-D-cystein (1) mit folgender Primärstruktur:

meso-Lanthionin

```
        CH2 ——————— S ——————— CH2
          |                     |
H-Ile-Ala-NH-CH-CO-Lys-Phe-Ile-NH-CH-CO—
          (S)                   (R)
```

```
    CH3        ß-Methyllanthionin
      |
(S)CH——————— S ——————— CH2
      |                  |
 NH-CH-CO-Pro-Gly-HN-CH-CO-Ala-Lys——————
   (S)                (R)
```

```
    CH3        meso-Lanthionin
     |
    CH        CH2 ——————— S ——————————————— CH2
     ||        |                 (S)         |          (Z)
 HN-C-CO-Gly-HN-CH-CO-Phe-Asn-HN-CH-CO-Tyr-HN-CH-CO-NH-CH
   (Z)          (S)              |           (R)        ||
                                CH2 ——————— S ——————— CH
```

S-(2-Aminovinyl)-D-cystein

wobei die dem N-Terminus näherstehenden Hälften der einzelnen Thioetheraminosäuren jeweils D-konfiguriert sind.

2. Epidermin gemäß Anspruch 1, gekennzeichnet durch folgende weitere Parameter:

1. Natur: farbloses Pulver;

2. Löslichkeit: Sehr gut löslich in Mischungen aus Wasser/Eisessig bzw. Methanol/Eisessig, löslich in niederen Alkoholen, unlöslich in Chloroform, Aceton, Diethylether, Petrolether;

3. Farbreaktion auf Kieselgelplatten: Ninhydrin, Chlor/TDM (TDM = 4,4'-Bis-(dimethylamino)-diphenylmethan), Orcin/Schwefelsäure, Anisaldehyd/Schwefelsäure. Zerstörungsfreier Nachweis im UV-Licht bei 254 nm und durch Sprühen mit Wasser;

4. Dünnschichtchromatographie: Es wurden Kieselgelfertigplatten 60 $F_{254}$ (Merck) verwendet.

System A: Chloroform/Methanol/17% Ammoniak (2/2/1),     RF = 0,73
System B: Chloroform/Methanol/17% Ammoniak (70/35/10)   RF = 0,30
System C: n-Butanol/Eisessig/Wasser (4/1/1)             RF = 0,05

5. HPLC : siehe Abb. 7;

6. Stabilität: Stabil von pH = 2 bis pH = 7, bei höheren pH-Werten tritt starker Aktivitätsabfall ein;

7. Molmasse: im Bereich von 2160 (ohne Anionen);

8. Ultraviolettabsorptionsspektrum: In wässriger Lösung langwelliges Maximum bei 267 nm (Abb. 3);

9. Infrarotabsorptionsspektrum: IR-Spektrum einer Probe in einer Kaliumbromidtablette (Abb. 4);

10. Kernmagnetische Resonanzspektren $^1$H-NMR Spektrum: Abb. 5, $^{13}$C-NMR-Spektrum: Abb. 6

3. Verfahren zur Herstellung, Isolierung und Reinigung des Antibiotikums Epidermin, gemäß Anspruch 1 und 2, dadurch gekennzeichnet, daß Staphylococcus epidermidis DSM 3095 aerobisch bei 34 bis 37°C in einer komplexen Nährlösung, bestehend aus 2 bis 4% einer Stickstoffquelle wie Fleischextrakt, 1 bis 3% eines Zuckers bzw. Zuckeralkohols als Kohlenstoffquelle und 0,25 bis 1% eines Carbonats und/oder 0,25 bis 0,5% eines Hydroxids eines Erdalkalimetalls, gezüchtet wird, die Kulturbrühe anschließend nach Entfernung der Zellen und anorganischer Salze zur Isolierung des gebildeten Wirkstoffes entweder mit n-Butanol bei pH 8 extrahiert, der Butanolextrakt evaporiert, der Rückstand in Methanol gelöst und die Lösung durch Etherfällung von den lipidischen Begleitstoffen befreit oder mit Polymeren auf Acrylester- oder Polystyrolbasis, behandelt wird, wobei das adsorbierte Epidermin mit Methanol/konzentrierte Salzsäure (99:1) vom Harz gelöst, die Lösung anschließend mit Ammoniak neutralisiert und am Vakuum eingeengt wird, und die so gewonnenen Isolate zur Abtrennung von weiteren niedermolekularen Peptiden, Aminosäuren und Salzen gelchromatographiert (Sephadex LH-20 mit Methanol/Essigsäure (95:5)) und anschließend einer multiplikativen Gegenstromverteilung nach Craig, bei welcher zuerst eine Flüssig-Flüssig-Verteilung mit dem System n-Butanol/Essigester/0,1 N Essigsäure (3:1:3) vorgenommen wird, bei der Epidermin am Start sitzen bleibt, und anschließend bei einer zweiten Verteilung mit dem neutralen System 2-Butanol/0,05 N Ammoniumacetat (1:1) die Isolierung des reinen Epidermins erreicht wird, unterworfen werden und aus dem Eluat durch Gefriertrocknung das Epidermin als farbloses Pulver isoliert wird.

4. Verfahren nach Anspruch 3, dadurch gekennzeichnet, daß als Stickstoffquelle 2 bis 4 Gew.-% eines Fleischextraktes, eines Gemisches aus allen 20 Aminosäuren in Aminosäure-Konzentrationen von jeweils 2 g/l, oder 2 bis 4 Gew.-% von Casaminoacid in Gegenwart von Tryptophan, als Kohlenstoffquelle 1 bis 3 Gew.-% Malzextrakt, Maltose, Galaktose, Laktose, Mannit, Glucose, Glycerin oder Kombinationen von Laktose mit Maltose, Galaktose mit Maltose, desweiteren 0,25 bis 1 Gew.-% Calciumcarbonat oder 0,25 bis 0,5 Gew.-% Calciumhydroxid in das Fermentationsmedium eingebracht und bei Verwendung von Casaminoacid noch Vitamine wie Biotin, Nicotinsäure, Thiamin, PyridoxinxHCl und Calciumpantothenat zugefügt werden, der pH-Wert vor der Fermentation auf 6,0 bis 7,0 eingestellt und der Produktionsverlauf durch laufende Probenahmen überprüft wird.

5. Epidermin erhalten nach dem Verfahren des Anspruchs 3.

6. Als neuer Epidermin-erzeugender und gegen Epidermin resistenter Stamm Staphylococcus epidermidis DSM 3095.

7. Staphylococcus epidermidids DSM 3095 mit folgender Charakterisierung

Gramfärbung :              positiv
Zellgröße :                Durchmesser 0,5-0,8 $\mu$m

| Koloniengröße : | ca. 1 mm Durchmesser |
| Aussehen der Kolonien : | glatt, glänzend, in der Mitte leicht erhöht |
| Kolonienfarbe : | gräulich - weiß |
| Zellen in Kultur : | oft Einzelzellen oder Zweier-Pakete, selten größere Haufen |
| Hämolyse : | auf Blutplatten ausgestrichene Kulturen zeigten starke Hämolyse |
| Anaerobes Wachstum : | der Stamm wächst auch unter anaeroben Bedingungen |
| Lysozymempfindlichkeit: : | die Zellen sind bis 2 mg/ml resistent gegen Lysozym |
| Lysostaphinempfindlichkeit : | die Zellen sind schon bei 20 μg/ml sensitiv für Lysostaphin |
| Epidermin-Resistenz : | bis 1 mg/ml nachgewiesen |
| Sonstige Resistenzen : | in Flüssigkultur zeigt der Stamm eine ausgeprägte Resistenz gegenüber Streptomycin (bis 1 mg/ml) und Spectinomycin (0,5 mg/ml) |
| Erhöhter NaCl-Gehalt : | Der Stamm wächst noch gut bis 15% Gew.-% Natriumchlorid, |

wobei die Kolonien oder ausgestrichenen Kulturen klebrig sind.

8. Antibakterielle Arzneimittelzubereitungen gekennzeichnet durch einen Gehalt an Epidermin gemäß Anspruch 1 und 2 neben üblichen Träger und/oder Hilfsstoffen.

9. Epidermin zur Verwendung als antibakterielles Mittel.

## Claims

1. Antibiotically active polypeptide known as epidermin, characterised by the following amino acid composition: Asn (1), Pro (1), Gly (2), Ala (2), Ile (2), Phe (2), Lys (2), Lan (2), β-Me-Lan (1), Dhb (1), Tyr (1), S-(2-aminovinyl)-D-cysteine (1) with the following primary structure:

23

meso-Lanthionine

ß-Methyllanthionine

meso-Lanthionine

S-(2-Aminovinyl)-D-cysteine

whilst the halves of the individual thioetheramino acids closer to the N-terminus are of D configuration.

2. Epidermin as claimed in claim 1, characterised by the following additional parameters:
   1. Nature: colourless powder;
   2. Solubility: very readily soluble in mixtures of water/glacial acetic acid or methanol/glacial acetic acid, soluble in lower alcohols, insoluble in chloroform, acetone, diethyl ether, petroleum ether;
   3. Colour reaction on silica gel plates: Ninhydrin, chlorine/TDM [TDM = 4,4'-bis-(dimethylamino)-diphenylmethane], orcin/sulphuric acid, anisaldehyde/sulphuric acid. Non-destructive detection in UV light at 254 nm and by spraying with water;
   4. Thin layer chromatography: ready-made silica gel plates 60 $F_{254}$ (Merck) were used.
      System A: chloroform/methanol/17% ammonia (2/2/1),     $R_F$ = 0.73
      System B: chloroform/methanol/17% ammonia (70/35/10)   $R_F$ = 0.30
      System C: n-butanol/glacial acetic acid/water (4/1/1)   $R_F$ = 0.05
   5. HPLC: see Fig. 7.
   6. Stability: stable from pH 2 to pH 7, but a sharp drop in activity occurs at higher pH values;
   7. Molecular mass: in the region of 2160 (without anions);
   8. Ultra-violet absorption spectrum: in aqueous solution, long-wave maximum at 267 nm (Fig. 3);
   9. Infra-red absorption spectrum: IR spectrum of a sample in a potassium bromide tablet (Fig. 4);
   10. Nuclear magnetic resonance spectra $^1$H-NMR spectrum: Fig. 5, $^{13}$C-NMR spectrum: Fig. 6.

3. Process for preparing, isolating and purifying the antibiotic epidermin, as claimed in claims 1 and 2, characterised in that Staphylococcus epidermidis DSM 3095 is grown aerobically at 34-37°C in a complex nutrient solution consisting of 2-4% of a nitrogen source such as meat extract, 1-3% of a sugar or sugar alcohol as the source of carbon and 0.25 to 1% of a carbonate and/or 0.25 to 0.5% of a hydroxide of an alkaline earth metal, then, after removal of the cells and inorganic salts, in order to isolate the active substance formed, the culture liquor is either extracted with n-butanol at pH 8, the butanol extract is evaporated, the residue is dissolved in methanol and the solution is freed from lipid contaminants by ether precipitation or it is treated with polymers based on acrylate or polystyrene, whilst the adsorbed epidermin is released from the resin with methanol/concentrated hydrochloric acid (99:1), the solution is then neutralised with ammonia and concentrated by evaporation in vacuo, and the isolated material thus obtained is subjected to gel chromatography [Sephadex LH-20 with methanol/acetic acid (95:5)] in order to separate off other low-molecular weight peptides, amino acids and salts, and then subjected to multiplicative countercurrent distribution according to Craig, in which first a liquid-liquid distribution is effected using a system of n-butanol/ethyl acetate/0.1 N acetic acid (3:1:3) in which epidermin is left on the baseline, and subsequently in a second distribution using the neutral system 2-butanol/0.05 N ammonium acetate (1:1), the pure epidermin is isolated as a colourless powder from the eluate by freeze-drying.

4. Process as claimed in claim 3, characterised in that, as nitrogen source, 2-4% by weight of a meat extract, a mixture of all 20 amino acids in amino acid concentrations of 2 g/l, or 2 to 4% by weight of casamino acid in the presence of tryptophan, and as carbon source 1 to 3% by weight of malt extract, maltose, galactose, lactose, mannitol, glucose, glycerol or combinations of lactose with maltose, or galactose with maltose, and 0.25 to 1% by weight of calcium carbonate or 0.25 to 0.5% by weight of calcium hydroxide are introduced into the fermentation medium and, if casamino acid is used, vitamins such as biotin, nicotinic acid, thiamine, pyridoxine.HCl and calcium pantothenate are added, the pH value is adjusted to 6.0 to 7.0 before fermentation and the course of production is monitored by continuous sampling.

5. Epidermin obtained by the process claimed in claim 3.

6. As a new epidermin-producing strain which is resistant to epidermin, Staphylococcus epidermidis DSM 3095.

7. Staphylococcus epidermidis DSM 3095 with the following characteristics:

| | |
|---|---|
| Gram coloration : | positive |
| Cell size : | 0.5-0.8 $\mu$m in diameter |
| Colony size : | about 1 mm in diameter |
| Appearance of colonies : | smooth, glossy, slightly raised in the centre |
| Colour of colonies : | greyish-white |
| Cells in culture : | often single cells or groups of two, seldom larger clumps |
| Haemolysis : | cultures spread on blood slides showed strong haemolysis |
| Anaerobic growth : | the strain grows even under anaerobic conditions |
| Lysozyme sensitivity : | the cells are resistant to lysozyme in quantities of up to 2 mg/ml |
| Lysostaphin sensitivity : | the cells are sensitive to lysostaphin even in quantities of 20 $\mu$g/ml |
| Epidermin resistance : | demonstrated up to 1 mg/ml |
| Other resistances : | in liquid culture the strain shows a marked resistance to Streptomycin (up to 1 mg/ml) and Spectinomycin (0.5 mg/ml) |
| Increased NaCl content : | the strain still grows well with a sodium chloride content of up to 15% by weight, |

the colonies or the spread-out cultures being adhesive.

8. Antibacterial pharmaceutical preparations, characterised in that they contain epidermin as claimed in claims 1 and 2 together with conventional carriers and/or excipients.

9. Epidermin for use as an antibacterial agent.

**Revendications**

1. Polypeptide à activité antibiotique, dénommé épidermine, caractérisé par la composition suivante: Asn (1), Pro (1), Gly (2), Ala (2), Ile (2), Phe (2), Lys (2), Lan (2), $\beta$-Me-Lan (1), Dhb (1), Tyr (1),S-(2-Aminovinyl)-D-cystéine (1) avec la structure primaire suivante:

méso-lanthionine

$$CH_2 \text{——} S \text{——} CH_2$$

H-Ile-Ala-NH-CH-CO-Lys-Phe-Ile-NH-CH-CO——

(S)                                    (R)

$$CH_3 \quad \beta\text{-méthyllanthionine}$$

(S) CH ——— S ——— $CH_2$

-NH-CH-CO-Pro-Gly-NH-CH-CO-Ala-Lys——

(S)                         (R)

$$CH_3 \quad \text{méso-lanthionine}$$

CH          $CH_2$ ——— S ——— $CH_2$

                        (S)                (Z)

-HN-C-CO-Gly-HN-CH-CO-Phe-Asn-HN-CH-CO-Tyr-HN-CH-CO-NH-CH

(Z)          (S)                          (R)

$$CH_2 \text{——} S \text{——} CH$$

S-(2-aminovinyl)-D-cystéine

dans lequel les moitiés des différents acides thioétheraminés les plus proches de l'extrémité N sont chacune de configuration D.

2.  Epidermine selon la revendication 1, caractérisée par les paramètres supplémentaires suivants:
1. nature: poudre incolore;
2. solubilité: très soluble dans des mélanges eau/acide acétique glacial et méthanol/acide acétique glacial, soluble dans les alcools inférieurs, insoluble dans le chloroforme, l'acétone, le diéthyléther, l'éther de pétrole;
3. réaction colorée sur des plaques de gel de silice: ninhydrine, chlore/TDM (TDM = 4,4'-bis-(diméthylamino)diphénylméthane), orcine/acide sulfurique, anisaldéhyde/acide sulfurique. Mise en évidence non destructive à la lumière UV à 254 nm et par aspersion d'eau;
4. chromatographie en couche mince: utilisation de plaques de gel de silice 60 $F_{254}$ (Merck) prêtes à l'emploi.

Système A: chloroforme/méthanol/ammoniaque à 17% (2/2/1),     RF = 0,73
système B: Chloroforme/méthanol/ammoniaque à 17% (70/35/10)     RF = 0,30
système C: n-butanol/acide acétique glacial/eau (4/1/1)     RF = 0,05

5. CLHP: voir figure 7;
6. stabilité: stable de pH = 2 à pH = 7, aux pH supérieurs apparition d'une forte chute de l'activité;
7. masse molaire: dans le domaine de 2160 (sans anions);
8. spectre d'absorption ultraviolet: en solution aqueuse maximum aux grandes longueurs d'onde à 267 nm (figure 3);
9. spectre d'absorption infrarouge: spectre IR d'un échantillon dans une pastille de bromure de potassium (figure 4);
10. spectres de résonance magnétique nucléaire spectre RMN-$^1$H: figure 5, spectre RMN-$^{13}$C: figure 6.

3.  Procédé de préparation, isolement et purification de l'antibiotique épidermine selon les revendications 1

26

et 2, caractérisé en ce que Staphylococcus epidermidis DSM 3095 est cultivé par voie aérobie entre 34 et 37°C dans une solution nutritive complexe, consistant en 2 à 4% d'une source d'azote telle qu'un extrait de viande, 1 à 3% d'un sucre ou d'un alcool de sucre comme source de carbone et 0,25 à 1% d'un carbonate et/ou 0,25 à 0,5% d'un hydroxyde d'un métal alcalinoterreux, le bouillon de culture, après élimination des cellules et des sels minéraux, pour isoler la substance active formée, est ensuite soit extrait au n-butanol à pH 8, l'extrait au butanol est évaporé, le résidu est dissous dans le méthanol et la solution est débarrassée des impuretés lipidiques par précipitation à l'éther, soit traité avec des polymères à base d'acrylate ou de polystyrène, l'épidermine adsorbée étant séparée de la résine par dissolution dans un mélange méthanol/acide chlorhydrique concentré (99:1), la solution est ensuite neutralisée à l'ammoniaque et concentrée sous vide, et les isolats ainsi obtenus sont chromatographiés sur gel (Sephadex LH-20 avec un mélange méthanol/acide acétique (95:5)) pour séparer d'autres peptides de faible poids moléculaire, d'autres acides aminés et d'autres sels, puis soumis à une séparation multiplicative à contre-courant selon Craig, dans laquelle une séparation liquide-liquide avec le système n-butanol/acétate/acide acétique 0,1 N (3:1:3) est d'abord réalisée, dans laquelle l'épidermine reste au départ, puis dans une seconde séparation avec le système neutre butanol-2/acétate d'ammonium 0,05 N (1:1) l'isolement de l'épidermine pure est obtenu, et l'épidermine est isolée sous forme de poudre incolore à partir de l'éluat par lyophilisation.

4. Procédé selon la revendication 3, caractérisé en ce que l'on introduit dans le milieu de fermentation comme source d'azote 2 à 4% en poids d'un extrait de viande, d'un mélange de tous les 20 acides aminés en des concentrations en acides aminés de 2 g/l à chaque fois, ou 2 à 4% en poids de casaminoacide en présence de tryptophane, comme source de carbone 1 à 3% en poids d'extrait de malt, maltose, galactose, lactose, mannitol, glucose, glycérine ou des combinaisons de lactose avec du maltose, de galactose avec du maltose, en outre 0,25 à 1% en poids de carbonate de calcium ou 0,25 à 0,5% en poids d'hydroxyde de calcium, et lors de l'utilisation de casaminoacide, en outre des vitamines comme la biotine, l'acide nicotinique, la thiamine, la pyridoxinexHCl et le pantothénate de calcium, on règle le pH entre 6,0 et 7,0 avant la fermentation et on surveille le déroulement de la production par des prélèvements continus d'échantillons.

5. Epidermine obtenue par le procédé selon la revendication 3.

6. Staphylococcus epidermidis DSM 3095 en tant que nouvelle souche productrice d'épidermine et résistante à l'épidermine.

7. Staphylococcus epidermidis DSM 3095 avec la caractérisation suivante

| | |
|---|---|
| coloration Gram: | positive |
| taille des cellules: | diamètre 0,5-0,8 $\mu$m |
| taille des colonies: | diamètre d'environ 1 mm |
| aspect des colonies: | lisse, brillant, légèrement relevé au milieu |
| couleur des colonies: | blanc-grisâtre |
| cellules en culture: | souvent cellules isolées ou groupes de deux, amas plus importants rares |
| hémolyse: | les cultures étalées sur des plaques de sang ont présenté une forte hémolyse |
| croissance anaérobie: | la souche croît aussi dans des conditions anaérobies |
| sensibilité au lysozyme: | les cellules sont résistantes au lysozyme jusqu'à 2 mg/ml |
| sensibilité à la lysostaphine: | les cellules sont sensibles à la lysostaphine dès 20 $\mu$g/ml |
| résistance à l'épidermine: | mise en évidence jusqu'à 1 mg/ml |
| autres résistances: | en culture liquide la souche présente une résistance marquée à la streptomycine (jusqu'à 1 mg/ml) et à la spectinomycine (0,5 mg/ml) |
| teneur en NaCl élevée: | la souche croît encore bien jusqu'à 15% en poids de chlorure de sodium, |

les colonies ou cultures étalées étant collantes.

8. Préparations médicamenteuses antibactériennes caractérisées par une teneur en épidermine selon les revendications 1 et 2 en plus des véhicules et/ou adjuvants habituels.

9. Epidermine utilisable comme agent antibactérien.

Abbildung 1:

Abbildung 2:

Abbildung 4:

Abbildung 5:

Abbildung 8:

Abbildung 9:

```
                    ┌──────────────┐
                    │ Kulturmedium │
                    └──────┬───────┘
                           │           Abzentrifugieren (Zellen und Kalk)
                           ▼
                    ┌──────────────┐
                    │  Überstand   │
                    └──────┬───────┘
Extraktion mit n-Butanol  / \      Adsorption an XAD-8,
Etherfällung             /   \     Elution mit methanolischer HCl-Lösung
                        ▼     ▼
          ┌─────────────────┐  ┌──────────────────┐
          │ biologisch aktiver │ biologisch aktives │
          │ Niederschlag     │  │ Eluat            │
          └────────┬────────┘  └────────┬─────────┘
                    \     Gelchromatographie an     /
                     \    Sephadex LH 20;  MeOH/    /
                      \   Essigsäure (99/5)        /
                       ▼                          ▼
              ┌──────────────────────────────┐
              │ biologisch aktive Fraktionen │
              └──────────────┬───────────────┘
                             │    Craig-Verteilung I (n-Butanol/Essig-
                             │    ester/0.1N HAc = 3/1/3) 160 Stufen
                             ▼
                    ┌──────────────┐
                    │ Roh- Epidermin │
                    └──────┬───────┘
                           │       Craig-Verteilung II (sec.-Butanol/
                           │       0.05N NH4Ac = 1/1) 440 Stufen
                           ▼
                    ┌──────────────┐
                    │  Epidermin   │
                    └──────────────┘
```

Abbildung 13:

Abbildung 14:

EP 0 181 578 B1

Abbildung 15:

42